# EUROPEAN PATENT APPLICATION

(11) **EP 4 001 429 A1**
(43) Date of publication of application: **25.05.2022**
(21) Application number: 20207729.3
(22) Date of filing: 16.11.2020
(51) Int. Cl.: C12Q 1/6806, G01N 33/50

(54) **ANALYSIS OF CRISPR-CAS BINDING AND CLEAVAGE SITES FOLLOWED BY HIGH-THROUGHPUT SEQUENCING (ABC-SEQ)**

(71) Applicant: Antibodies-Online GmbH, 52072 Aachen (DE)
(72) Inventor: PELLENZ, Stefan, 6291 LL Vaals (NL)
(74) Representative: Bauer, Dirk

(57) **Abstract**

The present invention relates to a method for the analysis of binding and cleavage sites followed by high-throughput sequencing. This method is called "ABC-seq". The method is based on CUT&RUN (or CUT&Tag), originally developed for the detection of epigenetic marks, in combination with recombinant catalytically active or inactive Cas and a bioinformatics pipeline to identify off-site binding and off-site cleavage events in parallel.

SEQUENCE LISTING

<110> antibodies-online GmbH

<120> Analysis of CRISPR-Cas Binding and Cleavage sites followed by
high-throughput sequencing (ABC-seq)

<130> ZUWAG017

<140> not yet assigned
<141> not yet filed

<160> 5

<170> BiSSAP 1.3.6

<210> 1
<211> 33
<212> DNA
<213> artificial sequence

<220>
<223> Primer

<400> 1
tcgtcggcag cgtcagatgt gtataagaga cag                                    33

<210> 2
<211> 34
<212> DNA
<213> artificial sequence

<220>
<223> Primer

<400> 2
gtctcgtggg ctcggagatg tgtataagag acag                                   34

<210> 3
<211> 19
<212> DNA
<213> artificial sequence

<220>
<223> Primer

<400> 3
ctgtctctta tacacatct                                                    19

<210> 4
<211> 50
<212> DNA
<213> artificial sequence

<220>
<223> Primer

<400> 4
aatgatacgg cgaccaccga gatctacact cgtcggcagc gtcagatgtg          50

<210> 5
<211> 53
<212> DNA
<213> artificial sequence

<220>
<223> Primer

<220>
<221> misc_feature
<222> 25..32
<223> /function="8nt barcode"

<400> 5
caagcagaag acggcatacg agatnnnnnn nngtctcgtg ggctcggaga tgt          53

## Description

The present invention concerns a method that is based on using CUT&RUN or CUT&Tag to validate genome wide RNA guided endonuclease (RGEN) CRISPR-Cas binding and cleavage sites.

### Background of the Invention

### CRISPR (clustered regularly interspaced short palindromic repeats)

Type II CRISPR systems employ a single DNA CRISPR associated (Cas) endonuclease to recognize double-stranded DNA substrates and cleave each strand with a distinct nuclease domain. Target site recognition is guided by a short CRISPR RNA (crRNA) containing a variable spacer complementing the target DNA sequence (protospacer) and a short CRISPR repeat sequence. An additional small noncoding RNA, called the trans-activating crRNA (tracrRNA), base pairs with the repeat sequence in the crRNA to form a dual-RNA hybrid structure. The Cas apoenzyme (apo-Cas) binds DNA nonspecifically prior to the binding of the guide RNA. Upon binding of the dual-RNA guides the RNA-endonuclease complex undergoes a conformational change. The dual-RNA guides the mature ribonucleoprotein to cleave a DNA containing a complementary 20-nucleotide (nt) target sequence. The tracrRNA is required for crRNA maturation in type II systems.

Directly adjacent to the CRISPR DNA target sequence is a conserved protospacer adjacent motif (PAM) of 2-5bp. Instead of base specific interactions with the target DNA PAM the Cas endonuclease contacts the phosphate backbone, suggesting that PAM recognition by the endonuclease is based on sterical factors.

Engineered CRISPR-Cas systems generally include a synthetic chimeric guide RNA (gRNA or sgRNA) that combine the crRNA and tracrRNA into a single RNA transcript. The gRNA confers targeting specificity and serves as scaffold for the Cas endonuclease. This simplifies the system while retaining fully functional CRISPR-Cas-mediated sequence-specific DNA cleavage.

Unlike other DNA editing platform like meganucleases, zing-finger nucleases (ZFN), or transcription activator-like effector nucleases (TALENs), recognition of the intended DNA cleavage site by Cas endonucleases is determined by the readily modifiable 20nt gRNA. The Cas endonuclease remains inactive without the guide RNAs. DNA recognition is not based on protein structure, thus eliminating the need for protein engineering of DNA-recognition domains.

### Cas9 endonuclease structure

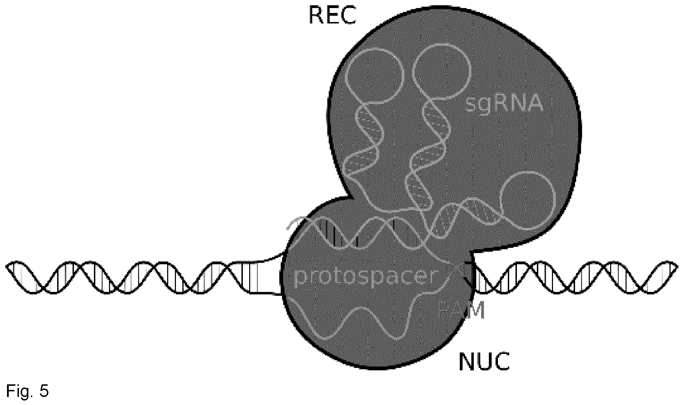

The most commonly used type II CRISPR Cas9 enzyme has a bipartite organization. The recognition (REC) lobe is characterized by three alpha-helical domains which are structurally rearranged upon loading of the gRNA. This rearrangement is essential for the Cas9 nuclease activity. The nuclease (NUC) lobe is composed of a RuvC nuclease domain and an HNH nuclease domain (see figure 5).

Each of these distinct nuclease domains selectively cleaves one strand of the DNA double helix. Introduction of the H840A or D10A mutations turns the *Streptococcus pyogenes* Cas9 SpCas) nuclease into a DNA nickase, i.e. it cleaves only one of the two DNA strands. Both mutations in conjunction render the Cas9 nuclease domain entirely inactive. However, the DNA binding specificity conferred by the REC domain and the guide RNA remains intact. This catalyctically inactive endonuclease is referred to as a "dead" Cas9 (dCas9).

Several other Cas9 proteins are in use, in particular *Staphylococcus aureus* (SaCas9) is frequently used because of its smaller size compared to SpCas9 for adeno-associated virus (AAV) lentiviral delivery systems.

### CRISPR-Cas12a (Cpf1)

The Type V Cas12a (formerly Cpf1) has an intrinsic RNase activity that allows it to process its own crRNA. This enables multiplexed DNA editing from a single RNA transcript. In contrast, Cas9-based gene editing relies on several guide RNAs being clones into one vector, rendering cloning complicated a facilitates undesired recombination events (Gier, R., Nature Comm. 11, No. 1 (2020), pp. 3455-3455; PMID 32661245). In addition, Cas12a has been used as a detector e.g. for viral ssDNA or RNA (Chen J., Science 360, No. 6387 (2018), pp. 436-439; PMID 29449511).

### CRISPR-Cas13a

Type VI CRISPR associated endonucleases like Cas13a and Cas13b recognize ssRNA rather than dsDNA. Using RNA CRISPR-Cas13a ribonucleoproteins in mammalian cells, knockdown levels have been attained comparable to RNAi, but with improved specificity. Similarly to Cas9 targeting DNA, it is also possible to take advantage of the catalytically inactive dPsp13b to specifically edit RNA. Cas13 is also being used to detect an ssRNA of interest. Binding of the ssRNA triggers its ssRNA cleavage activity. Cleavage of a quenched fluorescent ssRNA reporter results in a detectable signal (Gooetenberg, J., Science 365, No. 6336 (2017), pp. 438-442; PMID 28408723).

### CRISPR-Cas applications

DNA nucleases are often used in genome engineering to introduce a DNA double strand break (DSB) into the genomic DNA at a defined position. This DSB is subsequently repaired by the cell's own DNA repair machinery. Non-homologous end joining (NHEJ) of the generated DSB leads to error-prone repair. It is frequently used for targeted gene knock-outs through in-del open reading frame frameshift mutations or in-del mutations. In homology-directed repair (HDR) a repair template is used for a precise, non-mutagenic repair using the sister chromatid as a repair template. In genome engineering, this process is hijacked through the use of an artificial repair template containing a DNA sequence that is to be inserted into the genome surrounded by sequences that are homologous to the genomic target sequence. This way, HDR enables targeted gene insertions, corrections, conditional knock-outs, and other mutations.

Very recently a new method called "Prime Editing" has been described. It is based on CRISPR-Cas9 and appears to be able to efficiently and precisely install a wide range of sequences into DNA. Imperfect edits were almost entirely avoided (Anzalone, A. et al., Nature 576: 149-157 (2019); PMID 31634902).

The various aspects of CRISPR-Cas development and use are set forth in the following articles and patents/patent applications which are only a small selection of the available literature:
> "Multiplex genome engineering using CRISPR-Cas systems", Cong, L. et., Science, 339(6121):819-23 (2013);
> "RNA-guided editing of bacterial genomes using CRISPR-Cas systems", Jiang W. et al., Nat. Biotechnol. 31(3):233-9 (2013);
> "One-Step Generation of Mice Carrying Mutations in Multiple Genes by CRISPR-Cas-Mediated Genome Engineering", Wang H. et al., Cell 153(4):910-8 (2013);
> "Genome engineering using the CRISPR-Cas9 system", Ran, FA. et al., Nature Protocols 8(II):2281-308 (2013);
> "Development and Applications of CRISPR-Cas9 for Genome Engineering", Hsu, P et al., Cell 157(6):1262-78 (2014).
> "Genetic screens in human cells using the CRISPR-Cas9 system", Wang,T. et al., Science 343(6166): 80-84 (2014);
> "Rational design of highly active sgRNAs for CRISPR-Cas9-mediated gene inactivation", Doench JG et al., Nat. Biotechnol. 32(12): 1262-1267 (2014);
> "In vivo interrogation of gene function in the mammalian brain using CRISPR-Cas9", Swiech, L. et al., Nat. Biotechnol. 33(I): 102-106 (2015);
> "Genome-scale transcriptional activation by an engineered CRISPR-Cas9 complex", Konermann, S. et al., Nature. 517(7536):583-588 (2015).
> "A split-Cas9 architecture for inducible genome editing and transcription modulation", Zetsche B. et al., Nat. Biotechnol. 33(2): 139-142 (2015);
> "High-throughput functional genomics using CRISPR-Cas9", Shalem et al., Nature Reviews Genetics 16, 299-311 (2015).
> "Sequence determinants of improved CRISPR sgRNA design", Xu et al., Genome Research 25, 1147-1157 (2015).
> "A Genome-wide CRISPR Screen in Primary Immune Cells to Dissect Regulatory Networks," Parnas et al., Cell 162, 675-686 (2015).
> "Crystal Structure of Staphylococcus aureus Cas9," Nishimasu et al., Cell 162, 1113-1126 (2015)
> "RNA editing with CRISPR-Cas 13," Cox et al., Science. 358(6366): 1019-1027 (2017),
> "Programmable base editing of A-T to G-C in genomic DNA without DNA cleavage", Gaudelli et al., Nature 464(551); 464-471 (2017),
> "CRISPR-Cas9 Structures and Mechanisms", Jiang, F. & Doudna, J. A.,. Annu. Rev. Biophys. 46, 505-529 (2017),
> WO2014/093661
> WO2014/093694
> WO2014/093595
> WO2014/093635
> WO2014/093712
> WO2014/018423
> WO2014/204724
> WO2014/204726
> WO2014/204728
> WO2015/089364
> WO2015/089462
> WO2015/089465
> WO2015/058052
> WO2015/089364
> WO2015/089473
> WO2016/094872
> WO2016/094867
> WO2016/094874
> WO2016/106244
> WO2017/044893
> WO2017/075294
> WO2017/164936
> WO2019/005851.

### Problem to be solved

While genomic research has identified a number of genetic therapy targets that can modify the course of disease, there has been limited translation of genetic therapies into clinical use. Clustered regularly interspaced short palindromic repeats (CRISPR), a bacterial adaptive immune system, and its CRISPR-associated protein, e.g. Cas9, have gained attention for the ability to target and modify DNA sequences on demand with unprecedented flexibility and precision. The precision and programmability of the Cas proteins, e.g. Cas9, is derived from its complexation with a guide-RNA (gRNA) that is complementary to a desired genomic sequence. CRISPR systems open-up widespread applications including genetic disease modeling, functional screens, and synthetic gene regulation. The plausibility of in vivo genetic engineering using CRISPR has garnered significant traction as a next generation in vivo therapeutic. There are hurdles that need to be addressed before CRISPR-based strategies are fully implemented. At present, challenges associated with gene therapy techniques include unwanted immune system reactions, infection of incorrect cells, infection caused by the transfer agent, or the possibility of genes inserting into the wrong location, which has led to insertional oncogenesis in some cases. A particular concern is very low efficiency of CRISPR-mediated correction of genetic mutation using HDR in vivo. Such low efficiency limits the therapeutic use of CRISPR-mediated correction for most diseases. Accordingly, there remains a need in for methods of improving the efficiency of CRISPR-based gene editing and delivery for in vivo applications. For targeted knock-outs, error-prone NHEJ is a more efficient alternative.

Regardless of the repair pathway, DSBs represent a major threat to a genome's integrity and is paramount of the cell to repair this damage quickly. When DNA phosphate backbones are cleaved it is also possible that unforeseen repair events lead to the insertion of undesired DNA repair templates. If only one DNA strand is cut by a nickase, the DNA ends adjacent to the DSB remain physically connected. Therefore, initiation of HDR using a nickase is less likely to lead to the insertion of undesired DNA sequences. DNA nicking enzymes like a Cas9 nickase are interesting alternatives to endonucleases introducing DSBs for targeted genome engineering.

Alternatively, CRISPR interference (CRISPRi) and CRISPR activation (CRISPRa) using catalytically inactive dCas offer ways to regulate gene-expression transiently without permanent genome alterations. Similarly, dCas fusions to epigenetic modifiers enable epigenetic engineering.

A common factor for all CRISPR/Cas based applications is the need to reliably interact with the intended target site. Off-target effects can include small scale insertions/delections (indels) up to chromosomal translocations. Off-target binding sites are generally more numerous than binding sites. However, it is important to detect both and also to differentiate between binding and cleavage. Existing methods detect either off-site binding or off-site cleavage events.

### Disclosure of the Present Invention

The present invention presents a method for the analysis of binding and cleavage sites followed by high-throughput sequencing. This method is called "ABC-seq". This method is based on CUT&RUN (or CUT&Tag), originally developed for the detection of epigenetic marks, in combination with recombinant catalytically active or inactive Cas and a bioinformatics pipeline to identify off-site binding and off-site cleavage events in parallel. The method is exemplified with the commonly used SpCas9.

However, it can be readily adjusted to other Cas enzymes or even different endonclease platforms.The inventors of the present invention confronted with this afore-mentioned need have developed a method where the CUT&RUN (Cleavage Under Targets and Release Using Nuclease) process or CUT&Tag (Cleavage Under Targets and Tagmentation) process in combination with a dedicated bioinformatics pipeline is used to analyze CRISPR-Cas DNA binding and cleavage.

The present invention concerns a method as claimed in claim 1, 2, 4, 5, 6, 7, or 9 Preferred embodiments are the subject-matter of the dependent claims.

CUT&RUN offers a novel approach to pursue epigenetics (Skene, P. J. & Henikoff, S. An efficient targeted nuclease strategy for high-resolution mapping of DNA binding sites. Elife 6, 1-35 (2017); PMID 28079019 / Skene, P. J., Henikoff, J. G. & Henikoff, S., Targeted in situ genome-wide profiling with high efficiency for low cell numbers. Nat. Protoc. 13, 1006-1019 (2018); PMID 29651053). The method is designed to map genome wide transcription factor binding sites, chromatin-associated complexes, and histone variants and post-translational modifications (WO 2019/060907 A1). It is a relatively new method used to analyze protein interactions with DNA or RNA. CUT&RUN-sequencing combines antibody-targeted controlled cleavage by micrococcal nuclease with massively parallel sequencing to identify binding sites of DNA- or RNA-associated proteins.

CUT&RUN is performed in situ on immobilized, intact cells without crosslinking. DNA or RNA fragmentation is achieved using micrococcal nuclease that is fused to Protein A and/or Protein G (pAG-MNase). The fusion protein is directed to the desired target through binding of the Protein A/G moiety to the Fc region of an antibody bound to the target. DNA or RNA under the target is subsequently cleaved and released and the pAG-MNase-antibody-chromatin complex is free to diffuse out of the cell. DNA or RNA cleavage products are extracted and then processed by next generation sequencing (NGS) (Luo, D. et al., MNase, as a probe to study the sequence-dependent site exposures in the +1 nucleosomes of yeast. Nucleic Acids Res. 46, 7124-7137 (2018); PMID 29893974 / Chereji, R. V, Bryson, T. D. & Henikoff, S. Quantitative MNase-seq accurately maps nucleosome occupancy levels. Genome Biol. 20, 198 (2019); PMID 31519205).

ChIP (Chromatin Immunoprecipitation) has been the primary technique to map epigenetic markers for the last decades. More recently, ChIP followed by NGS (ChIP-seq) allows localization of epigenetic makers and protein binding sites on a genomic scale and has become a mainstay application to study gene regulation (Chereji, R., Genome Biology 20, No. 1 (2019), p. 198-198; PMID 29717046). However, in spite of the evolution of the readout the basic method to enrich the DNA of interest has remained unchanged - including its drawbacks.

CUT&RUN introduces some major modifications in order to eliminate some of the ChIP-seq shortcomings. Samples are not fixed, as it is the case for ChIP-seq, which can lead to epitope masking. Chromatin is fragmented in a targeted manner by a directed nuclease cleavage from intact cells reversibly permeabilized with the mild, nonionic detergent digitonin. The nuclear envelope remains intact since digitonin replaces cholesterol, which is only present in the plasma membrane. In contrast, chromatin for ChIP is prepared by sonication or enzymatic treatment of whole cells leading to a substantial background due to genomic DNA even after immunoprecipitation DNA enrichment. Because of this superior selectivity for chromatin containing the desired epitope, CUT&RUN has considerably lower background and better signal-to-noise ratio than ChIP-seq. This leads to a higher sensitivity and renders genomic features visible that are undetectable using ChIP-seq. In addition, less sequencing depth is required. Transcription factor binding sites can be mapped at bp resolution with 10⁶ reads. For abundant antigens such as H3K27me3 it is even possible to start with as few as 100 cells. Single-cell profiling using combinatorial indexing genomic analysis using CUT&RUN is possible since intact cells are being used (Cusanovich, D. A. et al. Multiplex single cell profiling of chromatin accessibility by combinatorial cellular indexing. Science 348, 910-4 (2015); PMID 25953818).

CUT&RUN, however, still has a characteristic feature that is carried over from ChIP-seq: the ends of the prepared DNA fragments need polishing and sequencing adapter ligation prior to the preparation of a sequencing library. A combination of the CUT&RUN protocol and tagmentation by a hyperactive Tn5 transposase resulted in the CUT&Tag (Cleavage Under Target and Tagmentation) method. Cells are immobilized on Concanavalin A beads and reversibly permeabilized using digitonin. Instead of the directed nuclease cleavage, however, DNA is fragmented by protein A and/or protein G fused transposase loaded with sequencing adapter duplexes. Sequencing adapters are attached to the DNA fragments directly during tagmentation. No further DNA end processing is necessary and the fragments can be used for sequencing library preparation. Reference is made to Nature Communication (Kaya Okur, H., Nature Comm. 10, No. 1 (2019), p. 1930; PMID 31036827)).

In contrast to other methods for the genome-wide mapping of chromatin accessibility improving upon ChIP-seq - e.g. DNase1 footprinting, MNase-seq, or ATAC-seq - CUT&RUN maps specific antigens or chromatin structure markers. Other tethering approaches like DNA adenine methyltransferase identification (DamID) and Chromatin Endogenous Cleavage (ChEC) also allow specific chromatin fragmentation depending on the protein of interest (Schmid et al., ChIC and ChEC: Genomic Mapping of Chromatin Proteins, Moll. Cell 16, 147-157 (2004); PMID 15469830).

Expression of recombinant fusion proteins does however limit the ChIP or ChEC scalability and they are not suitable to address specific histone modifications.

Chromatin Immunocleavage (ChIC) does also rely on a Protein A-MNase fusion protein that is tethered to an antibody against the protein of interest to direct DNA cleavage (Schmid et al., ChIC and ChEC: Genomic Mapping of Chromatin Proteins, Mol. Cell 16, 147-157 (2004); PMID 15469830). However, ChIC read-out is based on a Southern blot. Combination of ChIC on native cells or isolated nuclei immobilized on magnetic beads and high-throughput NGS gave rise to CUT&RUN.

The general CUT&RUN Protocol Steps are:
1. Optional Hypotonic Lysis to release Nuclei
2. Immobilize whole cells or nuclei with Magnetic Beads
3. Incubate with Antibody
4. Incubate with Protein A and/or Protein G MNase
5. Add Ca²⁺ (Reaction Start)
6. Add Chelator (Reaction Stop)
7. Pellet oligonucleosome
8. Sequencing

CUT&RUN advantages:
- Performed In situ on non-fixed cells; no chromatin fragmentation necessary.
- Low background and high sensitivity require low sequencing depth.
- Possible with low cell numbers down to 100 cells depending on the antigen.
- Simple, fast, amenable to automation.
- Accurate quantition by e.g. using heterologous spike-in DNA or carry-over E. coli DNA from pAG-MNase purification.

The general CUT&Tag Protocol Steps are:
1. Optional Hypotonic Lysis to release Nuclei
2. Immobilize whole cells or nuclei with Magnetic Beads
3. Incubate with Antibody
4. Incubate with a transposome.
5. Add Ca²⁺ (Reaction Start)
6. Add Chelator (Reaction Stop)
7. Pellet oligonucleosome
8. Sequencing

CUT&Tag advantages:
- Performed In situ on non-fixed cells; no chromatin fragmentation necessary.
- Low background and high sensitivity require low sequencing depth.
- No end-polishing and sequencing adapter ligation steps necessary.
- Possible with low cell numbers down to 100 cells depending on the antigen.
- Simple, fast, amenable to automation.
- Accurate quantitation by e.g. using heterologous spike-in DNA or carry-over E. *coli* DNA from the pAG-Tn5 purification.

Using CUT&RUN or CUT&Tag to validate CRISPR-Cas targeting

As used in this specification and the appended claims, the singular forms "a," "an," and "the" include plural references unless the context clearly dictates otherwise. Any reference to "or" herein is intended to encompass "and/or" unless otherwise stated.

The terms "comprising", "comprises" and "comprised of' as used herein are synonymous with "including", "includes" or "containing", "contains", and are inclusive or open-ended and do not exclude additional, non-recited members, elements, or method steps. The phraseology and terminology used herein is for the purpose of description and should not be regarded as limiting. The use of "including," "comprising," "having," "containing," "involving," and variations thereof, is meant to encompass the items listed thereafter and additional items. Use of ordinal terms such as "first," "second," "third," etc., in the claims to modify a claim element does not by itself connote any priority, precedence, or order of one claim element over another or the temporal order in which acts of a method are performed.

As used herein, the terms "synthetic" and "engineered" are used interchangeably and refer to the aspect of having been manipulated by the hand of man.

The terms "nucleic acid" and "nucleic acid molecule," as used herein, refer to a compound comprising a nucleobase and an acidic moiety, e.g., a nucleoside, a nucleotide, or a polymer of nucleotides. Typically, polymeric nucleic acids, e.g.,nucleic acid molecules comprising three or more nucleotides are linear molecules, n which adjacent nucleotides are linked to each other via a phosphodiester linkage. In some embodiments, "nucleic acid" refers to individual nucleic acid residues (e.g., nucleotides and/or nucleosides). In some embodiments, "nucleic acid" refers to an oligonucleotide chain comprising three or more individual nucleotide residues. As used herein, the terms "oligonucleotide" and "polynucleotide" can be used interchangeably to refer to a polymer of nucleotides (e.g., a string of at least three nucleotides). In some embodiments, "nucleic acid" encompasses RNA as well as single and/or double-stranded DNA. Nucleic acids may be naturally occurring, for example, in the context of a genome, a transcript, an mRNA, tRNA, rRNA, siRNA, snRNA, a plasmid, cosmid, chromosome, chromatid, or other naturally occurring nucleic acid molecule. On the other hand, a nucleic acid molecule may be a non-naturally occurring molecule, e.g., a recombinant DNA or RNA, an artificial chromosome, an engineered genome, or fragment thereof, or a synthetic DNA, RNA, DNA/RNA hybrid, or include non-naturally occurring nucleotides or nucleosides. Furthermore, the terms "nucleic acid," "DNA," "RNA," and/or similar terms include nucleic acid analogs, i.e., analogs having other than a phosphodiester backbone. Nucleic acids can be purified from natural sources, produced using recombinant expression systems and optionally purified, chemically synthesized, etc. Where appropriate, e.g., in the case of chemically synthesized molecules, nucleic acids can comprise nucleoside analogs such as analogs having chemically modified bases or sugars, and backbone modifications. A nucleic acid sequence is presented in the 5' to 3' direction unless otherwise indicated. In some embodiments, a nucleic acid is or comprises natural nucleosides (e.g., adenosine, thymidine, guanosine, cytidine, uridine, deoxyadenosine, deoxythymidine, deoxyguanosine, and deoxycytidine); nucleoside analogs (e.g., 2-aminoadenosine, 2- thiothymidine, pyrrolo-pyrimidine, 3-methyl adenosine, 5-methylcytidine, 2- aminoadenosine, C5-bromouridine, C5-fluorouridine, C5-iodouridine, C5-propynyl-uridine, CS-propynyl-cytidine, C5-methylcytidine, 2-aminoadenosine, 7-deazaadenosine, 7-deazaguanosine, 8-oxoadenosine, 8-oxoguanosine and 2-thiocytidine); chemically modified bases; biologically modified bases (e.g., methylated bases); intercalated bases; modified sugars (e.g., 2'-fluororibose, ribose, 2'-deoxyribose, arabinose, and hexose); and/or modified phosphate groups (e.g., phosphorothioates and 5'-N-phosphoramidite linkages).

The terms "protein," "peptide," and "polypeptide" are used interchangeably herein and refer to a polymer of amino acid residues linked together by peptide (amide) bonds. The terms refer to a protein, peptide, or polypeptide of any size, structure, or function. Typically, a protein, peptide, or polypeptide will be at least three amino acids long. A protein, peptide, or polypeptide may refer to an individual protein or a collection of proteins. One or more of the amino acids in a protein, peptide, or polypeptide may be modified, for example, by the addition of a chemical entity such as a carbohydrate group, a hydroxyl group, a phosphate group, a farnesyl group, an isofarnesyl group, a fatty acid group, a linker for conjugation, functionalization, or other modification, etc. A protein, peptide, or polypeptide may also be a single molecule or may be a multi-molecular complex.

A protein, peptide, or polypeptide may be just a fragment of a naturally occurring protein or peptide. A protein, peptide, or polypeptide may be naturally occurring, recombinant, or synthetic, or any combination thereof. A protein may comprise different domains, for example, a nucleic acid binding domain and a nucleic acid cleavage domain. In some embodiments, a protein comprises a proteinaceous part, e.g., an amino acid sequence constituting a nucleic acid binding domain, and an organic compound, e.g., a compound that can act as a nucleic acid cleavage agent.

As used herein, "modifying" ("modify") one or more target nucleic acid sequences refers to changing all or a portion of a (one or more) target nucleic acid sequence and includes the cleavage, introduction (insertion), replacement, and/or deletion (removal) of all or a portion of a target nucleic acid sequence. All or a portion of a target nucleic acid sequence can be completely or partially modified using the methods provided herein. For example, modifying a target nucleic acid sequence includes replacing all or a portion of a target nucleic acid sequence with one or more nucleotides (e.g., an exogenous nucleic acid sequence) or removing or deleting all or a portion (e.g., one or more nucleotides) of a target nucleic acid sequence. Modifying the one or more target nucleic acid sequences also includes introducing or inserting one or more nucleotides (e.g., an exogenous sequence) into (within) one or more target nucleic acid sequences.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The terms "Protein A-MNase", "Protein G-MNase", "Protein A-protein G-MNase", "pA-MNase", "pG-MNase", and "pAG-Mnase" are used interchangeably herein and refer to a recombinant micrococcal nuclease-protein A, micrococcal nuclease-protein G, or micrococcal nuclease-protein A-protein G fusion protein.

The terms "Protein A-Tn5", "Protein G-Tn5", "Protein A-protein G-Tn5", "pA-Tn5", "pG-Tn5", and "pAG-Tn5" are used interchangeably herein and refer to a recombinant hyperactive transposase 5-protein A, hyperactive transposase 5-protein G, or hyperactive transposase 5-protein A-protein G fusion protein. The term "transposome" refers to a protein A and/or protein G-Tn5 loaded with oligonucleotide duplex adapters high-throughput sequencing

In general, a CRISPR-Cas or CRISPR system as used in herein and in documents, such as WO 2014/093622 (PCT/US2013/074667), refers collectively to transcripts and other elements involved in the expression of or directing the activity of CRISPR-associated ("Cas") genes, including sequences encoding a Cas gene, a traer (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas, such as Cas9, e.g. CRISPR RNA and transactivating RNA (tracrRNA) or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system).

A protospacer adjacent motif (PAM) or PAM-like motif directs binding of the effector protein complex to the target locus of interest. The PAM may be a 5' PAM (i.e., located upstream of the 5' end of the protospacer) or a 3' PAM (i.e., located downstream of the 5' end of the protospacer). The term "PAM" may be used interchangeably with the term "PFS" or "protospacer flanking site" or "protospacer flanking sequence".

In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise DNA or RNA polynucleotides. The term "target DNA or RNA" refers to a DNA or RNA polynucleotide being or comprising the target sequence. In other words, the target RNA may be a polynucleotide or a part of a polynucleotide to which a part of the gRNA, i.e. the guide sequence, is designed to have complementarity and to which the effector function mediated by the complex comprising CRISPR effector protein and a gRNA is to be directed. In some embodiments, a target sequence is located in the nucleus or cytoplasm of a cell.

Non-limiting examples of Cas proteins include CasI, CasIB, Cas2, Cas3, Cas4, Cas5, Cas6, Cas7, Cas8, Cas9 (also known as Csnl and CsxI2), Cas12 or Cas13. According to the present invention Class 2 subtypes II, V, and VI SpCas9, SaCas9, Cas12a/Cpf1 and Cas13 are preferred.

As described above, CRISPR-Cas based nucleic acid manipulation opens many venues for any applications ranging from basic research to gene therapy and genome engineering because of the great flexibility of the system in terms of binding specificity and functionality inherent to the CRISPR-Cas nucleoproteins themselves or endowed by possible fusion proteins (Jiang, F. & Doudna, J. A., CRISPR-Cas9 Structures and Mechanisms. Annu. Rev. Biophys. 46, 505-529 (2017); PMID 28375731). For any of these applications, reducing or entirely avoiding any unwanted off-target effects at sites with sequence homology to the targeted sites is paramount.

Engineered DNA-binding molecule-mediated chromatin immunoprecipitation (enChIP) does already allow mapping of dCas, e.g. dCas9, binding sites. However, the readout for enChIP is either qPCR or NGS. Monitoring of enChIP enrichment of dCas9 binding sites is restricted to known binding sites due to the selection of specific PCR oligonucleotide primers. Accordingly, the outcome of the experiment is biased. NGS on the other hand does contain a considerable amount of background compared to CUT&RUN.

Combining binding of a catalytically inactive dCas with CUT&RUN or CUT&Tag allows precise mapping of CRISPR-Cas binding sites with minimal background. Unlike enChIP followed by qPCR the approach is not biases and allows a comprehensive coverage of dCas binding sites. Like enChIP followed by NGS the reduced background signal will allow mapping of genomic binding sites to which the dCas binds strongly. In addition, dCas binding following by CUT&RUN is expected to also reveal less favorable binding sites, e.g. because of nucleotide mismatches with the PAM or the protospacer itself. Sites that are bound less frequently might remain undetected using enChIP followed by NGS but are still of high interest, e.g. if they are situated in regulatory sequences of and oncogene.

Localization of the pAG-MNase or pAG-Tn5 in the vicinity of the dCas binding sites can be achieved either using an antibody specific for the dCas used. Alternatively, a dCas containing a protein tag such as a FLAG-tag can be used in conjunction with a antibody against this tag.

The inventors are aware that Cas with two inactive nuclease domains in the NUC lobe, like dCas, no longer initiates DNA-repair through insertion of DSBs but it still binds DNA or RNA specifically. A fusion of such a protein to a protein or protein domain with a particular activity allows targeted manipulation of genomic loci. Binding of catalytically inactive dCas to transcription start sites have been shown to repress transcription by blocking the transcription initiation site. This CRISPRi can also be achieved by fusing transcriptional repressor domains such as the Kruppel associated box (KRAB) domain to a dCas unit. Likewise, specific genes can be activated using CRISPRa employing dCas and transcriptional activators such as Vp64.

Base editors are fusions of a dead CRISPR-dCas and cytosine base editors (CBE) or adenine base editors (ABE). CBEs like APOBEC convert cytidine to uridine which is subsequently converted to thymidine by the cell's base excision repair (BER) mechanism. The results is a cytidine to thymidine transition and adenine to guanine respectively for the opposite DNA strand. Engineered ABEs convert adenosine to inosine, thus creating an adenine to guanine transition and thymidine to cytidine on the opposite strand. Manipulation of specific bases using base editors is generally higher than genomic modifications using HDR.

Similar to enabling CRISPRi and CRISPRa fusions of CRISPR-dCas with epigenetic modifiers like histone acyltransferases, methyltransferases, or enyzmes involved in DNA de-/methylation enables targeted manipulation of epigenetic marks. It is such possible to introduce inheritable gene expression markers unlike termporary CRISPRi and CRISPRa without the need to generate DSBs.

These are just a few of the possible applications for CRISPR-Cas. Catalytically active CRISPR-Cas, nickases, or dead nucleases can be used in virtually any application relying on a site specific interaction with DNA. Because of the ease to alter DNA binding specificity by using different gRNA the system is extremely flexible. In addition, various gRNAs can be used at the same time in order to target various genomic sites simultaneously. The "multiplexing" allows e.g. editing various genomic sites at once or the deletion of larger regions by removing sequences between two gRNA target sites.

### Direct or indirect CUT&RUN

An antibody specific for the protein of interest is crucial to direct the pAG-MNase mediated nucleic acid cleavage to the intended site. The Protein A/G portion tethers the fusion protein to the Fc region of the antibody bound to its antigen. This allows the pAG-MNase nuclease portion to cleave the nucleic acid under the targeted protein and to release the nucleic acid.

Depending on the host species and isotype of the antibody and the Protein A and/or Protein G MNase fusion protein, it can be necessary to include a secondary antibody for pAG-MNase binding (Skene, P. J. & Henikoff, S. An efficient targeted nuclease strategy for high-resolution mapping of DNA binding sites. Elife 6, 1-35 (2017)). For example, if the pA-MNase is used in conjunction with a primary mouse IgG1 or goat IgG antibody, it has advantages to use a rabbit secondary antibody. Protein A binds well to rabbit or guinea pig IgG antibodies but only poorly to mouse IgG1 or goat IgG. No additional secondary antibody is needed for CUT&RUN when using pAG-MNase (Meers, M. P., Bryson, T. D., Henikoff, J. G. & Henikoff, S. Improved CUT&RUN chromatin profiling tools. Elife 8, e46314 (2019); PMID 31232687).

CUT&RUN Sets are commercially available from the company "antibodies-online GmbH", Aachen, Germany (www.antibodies-online.com).

The CUT&RUN Positive Control (e.g. Antibodies-online GmbH, #ABIN3023255) and CUT&RUN Negative Control (e.g. Antibodies-online GmbH, #ABIN6923140) are for assessing cleavage and chromatin release without the need to sequence the released DNA fragments. It is not recommended to use a no-antibody negative control: untethered pAG-MNase will non-specifically bind and cleave any accessible DNA, thus increasing background signal.

### Mapping of genome-wide CRISPR-Cas binding and cleavage sites: ABC-seq (analysis of binding and cleavage by next-generation sequencing)

As outlined above, both cleavage-competent and -incompetent CRISPR-Cas ribonucleoproteins have distinct applications. Depending on the application, target site binding or target-site cleavage is a requirement. CRISPR-Cas DNA binding is a much more frequent event than DNA cleavage. However, both options have to be quantitatively accounted for.

Various experimental methods exist to interrogate cleavage events indirectly by tracking changes introduced through DSB repair, e.g. CIRCLE-seq, Digenome-seq, GUIDE-seq. Other methods detect capture of defined DNA fragments in DSB sites trough linear amplification, e.g. BLISS, GUIDE-seq. These methods map CRISPR-Cas off-site cleavage events indirectly by detecting DSB repair products on the DNA level. DISCOVER-seq on the other hand tracks MRE11 recruitment sites as a correlate for DSBs: DNA bound to the MRE11 protein is enriched through ChIP, followed by high-throughput sequencing. All of these methods track cleavage events. They do not detect DNA binding events..

It should also be noted that some of these methods may pose problems which limit their applicability. GUIDE-seq for example requires delivery of chemically modified double stranded oligonucleotides which can be detrimental to certain primary cells types and complicate its use in tissue samples. Digenome-seq is carried out in vitro on isolated genomic DNA and is consequently not suitable to identify off-target binding in situ.

In contrast, the aforementioned enChIP method does detect off-site binding of endonucleases expressed with protein tags such as a FLAG-tag. Bound DNA sequences are enriched via ChIP using a tag-specific antibody. The enChIP readout was originally qPCR of putative binding sites predicted by a computer-based algorithm based on certain assumptions regarding the protein's tolerance of base pair variations within its binding site. This shortcoming can be balanced by high-throughput sequencing readout of the enriched DNA sequences. However, enChIP can only detect binding sites, not cleavage sites, and it comes with ChIP-seq inherent issues such as the necessity for a high sequencing depth and low signal-to-noise ratio

### Description of Exemplary Embodiments

Disclosed herein is a method for detecting the binding of RGENs to genomic DNA in-situ in a cell or a population of cells. The present invention represents a major improvement compared to methods known in the art for mapping of genome wide interactions of RGENs such as CRISPR/Cas9 and other DNA modifying enzymes including but not limited to TALENs, ZFN, and meganucleases with chromatin in situ.

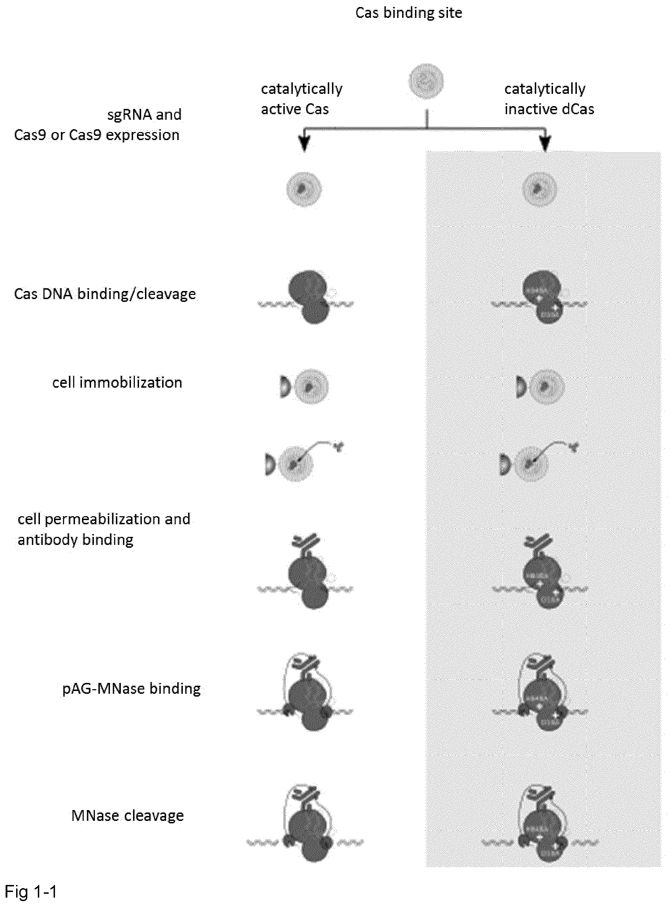

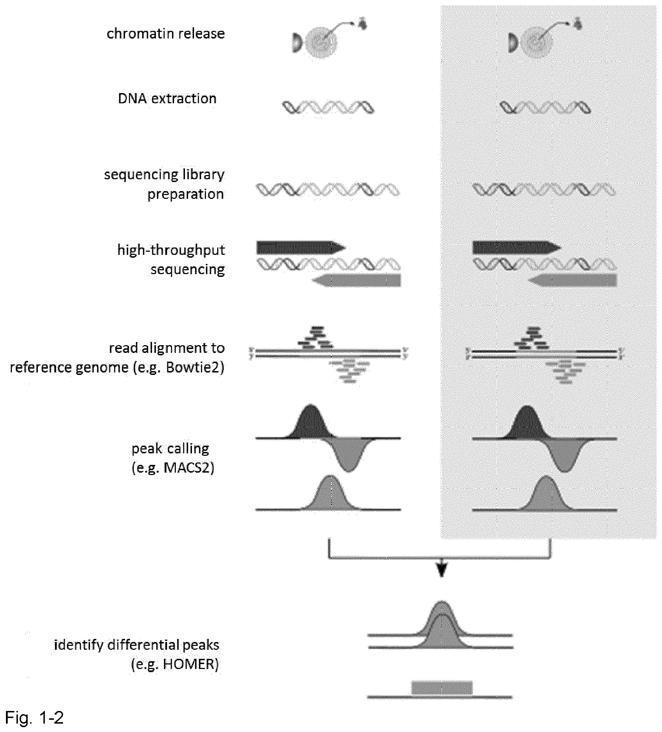

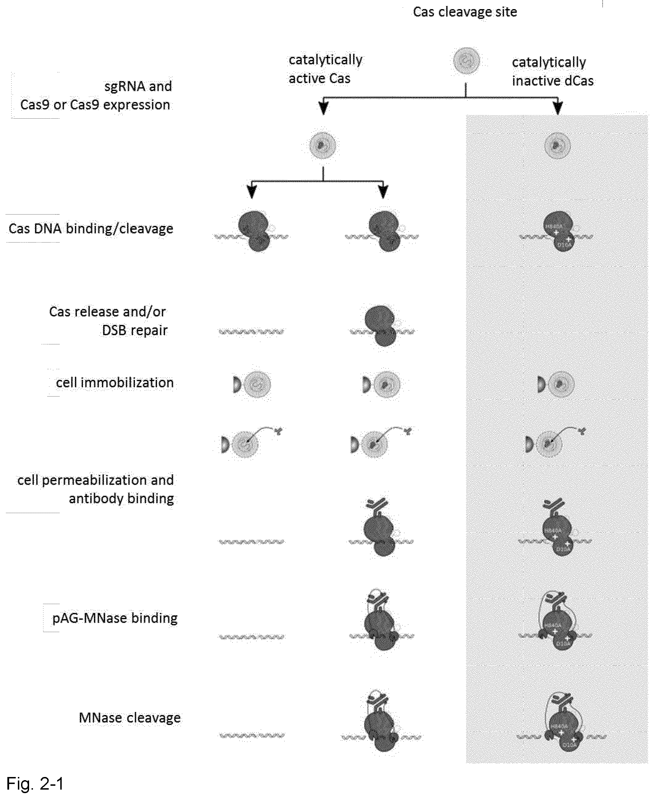

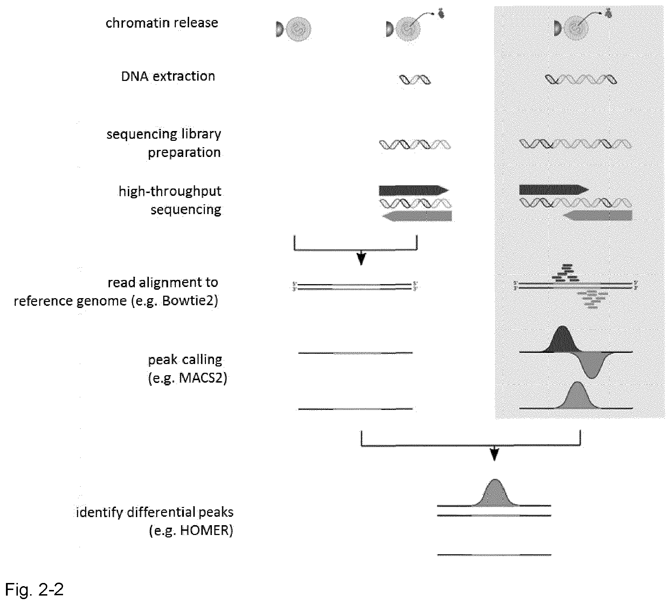

In certain embodiments, the interaction of the DNA modifying protein with the DNA component of chromatin is restricted to DNA binding. A catalytically inactive RGEN, e.g. CRISPR/dCas9, binds to its DNA substrate (Fig.1 right panel and Fig.2, right panel). Subsequently, the DNA binding sites are enriched using CUT&RUN. In short, cells are immobilized on a solid support, permeabilized using a mild detergent (e.g. digitonin), and an RGEN-specific immunoglobulin antibody binds the RGEN bound to its DNA substrate. Subsequently protein A and/or Protein G micrococcal nuclease fusion protein (pA/G-MNase) binds to the Fc fragment of the antibody via its protein A and/or protein G portion, thus tethering the MNase to the protein of interest prior to DNA fragmentation by the MNase. Upon initiation of MNase cleavage, complexes consisting of chromatin, the catalytically inactive RGEN, the antibody, and the pA/G-MNase are released and diffuse out of the cells. DNA is prepared from these complexes, converted into a sequencing library, and subjected to high-throughput sequencing. Sequencing reads are aligned to a reference genome and peaks corresponding to RGEN binding sites are identified.

In certain embodiments, the DNA modifying protein is a catalytically active RGEN; e.g. CRISPR/Cas9 (Fig.1 left panel and Fig.2, left panel). The RGEN binds to its DNA substrate and a subset of these bound sequences is then cleaved by the active endonuclease. Sequences that are bound but not cleaved and sequences that are cleaved and non-mutagenically repaired are identified using CUT&RUN. Sequences that are cleaved and mutagenized during the DSB repair are isolated using CUT&RUN and sequenced. Indels alter the DNA sequence so that it cannot be bound anymore by the RGEN. The corresponding sequencing reads do not align to the reference genome. Alternatively, the RGEN cleaves its DNA substrate once the DSB has been generated. Consequently, no sequencing reads are generated in this position. In both cases, peaks are missing at the positions of the DSB.

A comparison of the data generated with the catalytically inactive RGEN and the catalytically active RGEN reveals binding and cleavage sites: peaks that are present in both data sets correspond to binding sites. Peaks that are only present in the data set generated using the catalytically inactive RGEN correspond to cleavage sites. Thus, ABC-seq can detect both RGEN binding sites and cleavage sites. Methods known in the art are only suitable to detect either DNA binding sites or cleavage sites.

In other words, the present invention is based on the expression of an active and an inactive RGEN (e.g. CRISPR/Cas) with identical target sequence(s) in two parallel experiments: a binding and a binding/cleavage occurrence. The bioinformatic comparison of the identified sequencing peaks for the inactive (only binding) and the active Cas (binding and cleavage) provides a clear distinction since peaks which occur only in connection with the inactive enzyme but disappear when using the active enzyme are identified as cleavage sites.

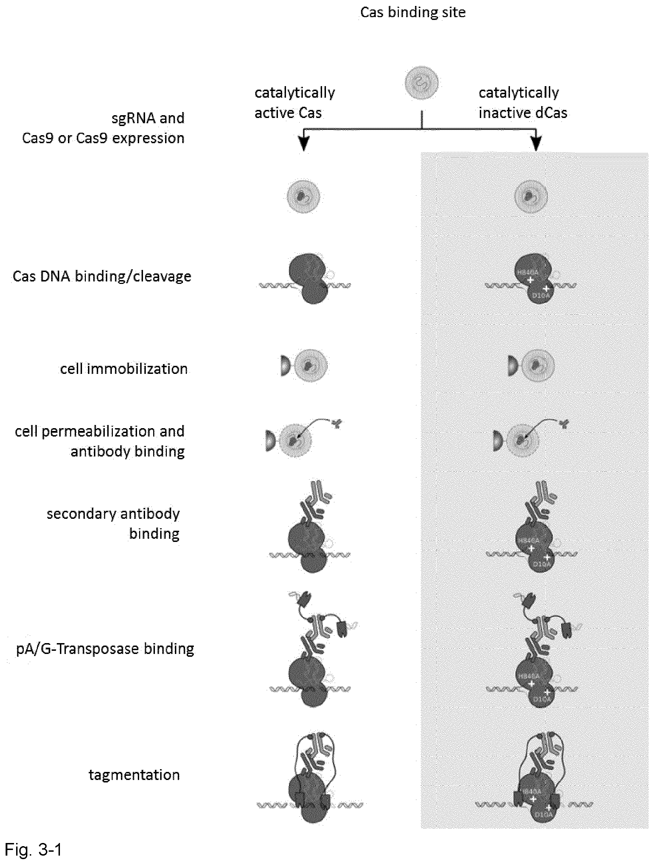

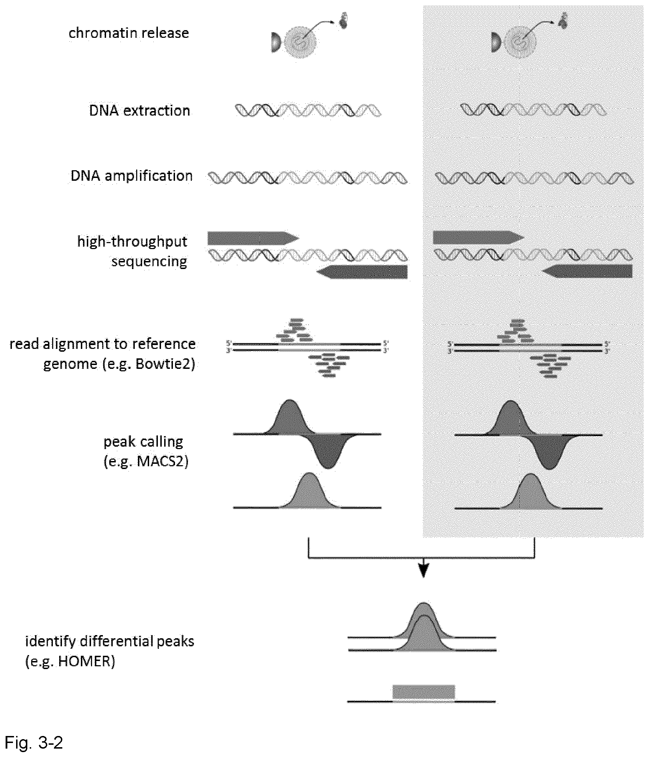

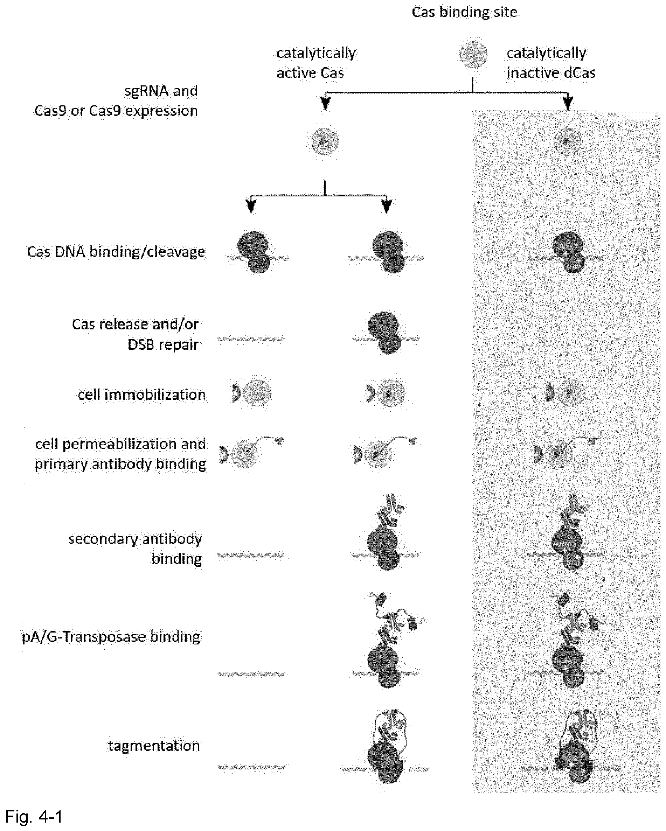

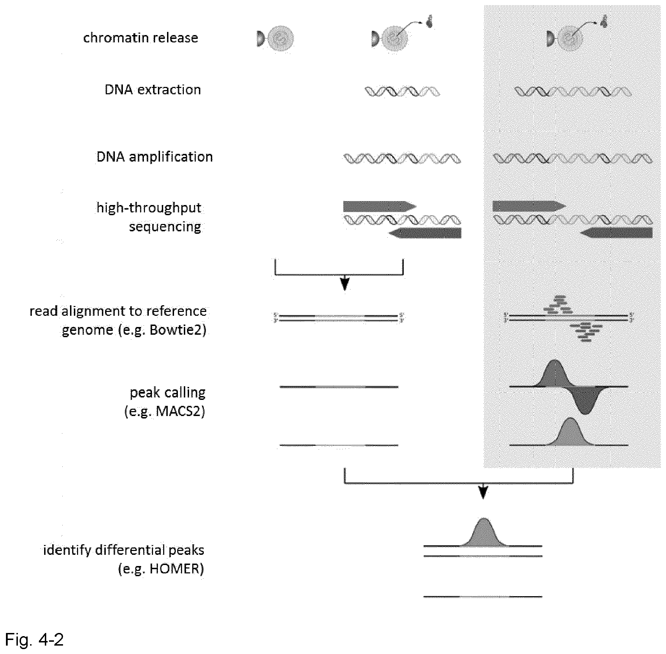

In certain embodiments, CUT&Tag is used for the enrichment of the DNA binding sites (Fig.3 right panel and Fig.4, right panel). In short, cells are immobilized on a solid support, permeabilized using a mild detergent (e.g. digitonin), and an RGEN-specific immunoglobulin antibody binds the RGEN bound to its DNA substrate. A secondary antibody binds to the first antibody. Subsequently a protein A and/or Protein G hyperactive transposase 5 (pA/G-Tn5) binds to the Fc fragments of the antibodies via its protein A and/or protein G portion, thus tethering the Tn5 to the protein of interest. Upon initiation of tagmentation, the transposome attaches the sequencing adapter to the DNA ends and complexes consisting of chromatin, the catalytically inactive RGEN, the antibody, and the pA/G-Tn5 are released and diffuse out of the cells. DNA is prepared from these complexes and subjected to high-throughput sequencing. Sequencing reads are aligned to a reference genome and peaks corresponding to RGEN binding sites are identified.

In certain embodiments, genome wide DNA cleavage sites of a catalytically active RGEN; e.g. CRISPR/Cas9 are enriched using CUT&Tag (Fig.3 left panel and Fig.4, left panel). The RGEN binds to its DNA substrate and a subset of these bound sequences is then cleaved by the active endonuclease. Sequences that are bound but not cleaved and sequences that are cleaved and non-mutagenically repaired are identified using CUT&RUN. Sequences that are cleaved and mutagenized during the DSB repair are isolated using CUT&RUN and sequenced. Indels alter the DNA sequence so that it cannot be bound anymore by the RGEN. The corresponding sequencing reads do not align to the reference genome. Alternatively, the RGEN cleaves its DNA substrate once the DSB has been generated. Consequently, no sequencing reads are generated in this position. In both cases, peaks are missing at the positions of the DSB.

In other embodiments, isolated nuclei or tissue samples can be used instead of cells as sample material.

In certain embodiments, the 3' repair exonuclease 2 (Trex2) is added simultaneously with the catalytically active RGEN to avoid a repeat target site cleavage and repair cycle. Trex2 has been shown to drive mutagenic DSB repair. Erroneous repair subsequently to Cas cleavage increases the number of sequencing reads in the position of a DSB that do not align with the reference genome, thus facilitating the identification of RGEN cleavage sites (Certo, M., Nature Methods 9, No. 10 (2012), pp. 973-975; PMID 22941364 / US 2016/0304855 A1).

In one embodiment, the RGEN and Trex2 are delivered simultaneously by lipid transfection or electroporation. In a different embodiment, both enzymes are simultaneously expressed ectopically from recombinant expression plasmids or co-expressed from the same expression plasmid.

The following examples are intended to illustrate various embodiments of the invention. As such, they are not to be understood as limitations on the scope of the invention. It will be apparent to one skilled in the art that various equivalents, changes, and modifications may be made without departing from the scope of invention, and it is understood that such equivalent embodiments are to be included herein.

Particularly, the present invention concerns a method to validate CRISPR-Cas targeting comprising the following steps:
(a) Expressing a catalytically inactive Cas protein (dCas) or catalytically active Cas protein and a single or several sgRNA in target cells,
(b) Optionally hypotonic lysis of the cells of step (a) to release nuclei,
(c) Immobilizing whole cells of step (a) or nuclei of step (b) with magnetic beads, preferably Concanvalin A beads,
(d) Incubating the product of step (c) with an anti-Cas IgG antibody, preferably rabbit anti-Cas IgG antibody,
(e) Optionally incubating the product of step (d) with a secondary IgG antibody, preferably anti-rabbit IgG antibody,
(f) Incubating the product of step (e) with ProteinA and/or ProteinG-MNase fusion protein (pAG-MNase),
(g) Adding of a Ca²⁺ ions-containing buffer to start MNase digestion and release of pAG-MNase-antibody-chromatin complexes,
(h) Adding of a chelator-containing buffer to stop the reaction of step (g),
(i) Pelletizing the obtained chromatin fragments and obtaining pAG-MNase-bound digested chromatin fragments from the supernatant,
(j) Extracting of DNA and RNA, respectively, from the chromatin fragments of step (i),
(k) High-throughput sequencing of DNA and RNA, respectively,
(l) Bioinformatic identification of differential peaks of sequencing reads in samples prepared using the catalytically inactive Cas protein (dCas) or catalytically active Cas proteins (Cas)

In an alternative embodiment, the present invention concerns a method to validate CRISPR-Cas targeting comprising the following steps:
(a) Expressing a catalytically inactive Cas protein (dCas) or catalytically active Cas proteins (Cas), a single or several sgRNA, and Trex2 in target cells,
(b) Optionally hypotonic lysis of the cells of step (a) to release nuclei,
(c) Immobilizing whole cells of step (a) or nuclei of step (b) with magnetic beads, preferably Concanvalin A beads
(d) Incubating the product of step (c) with an anti-Cas IgG antibody, preferably a rabbit anti-Cas IgG antibody
(e) Optionally incubating the product of step (d) with a secondary IgG antibody, preferably anti-rabbit IgG antibody
(f) Incubating the product of step (e) with ProteinA-ProteinG-MNase fusion protein (pAG-MNase),
(g) Adding of a Ca²⁺ ions-containing buffer to start MNase digestion and release of pAG-MNase-antibody-chromatin complexes,
(h) Adding of a chelator-containing buffer to stop the reaction of step (g),
(i) Pelletizing the obtained chromatin fragments and obtaining pAG-MNase-bound digested chromatin fragments from the supernatant,
(j) Extracting of DNA and RNA, respectively, from the chromatin fragments of step (i),
(k) High-throughput sequencing of DNA and RNA, respectively,
(l) Identification of differential peaks of sequencing reads in samples prepared using the catalytically inactive Cas protein (dCas) or catalytically active Cas proteins (Cas)

In an alternative embodiment, the present invention concerns a method to validate CRISPR-Cas targeting comprising the following steps:
(a) Expressing a catalytically inactive Cas protein (dCas) or catalytically active Cas protein without sgRNA,
(b) Optionally hypotonic lysis of the cells of step (a) to release nuclei,
(c) Immobilizing whole cells of step (a) or nuclei of step (b) with magnetic beads, preferably Concanvalin A beads,
(d) Incubating the product of step (c) with an anti-Cas IgG antibody, preferably rabbit anti-Cas IgG antibody,
(e) Optionally incubating the product of step (d) with a secondary IgG antibody, preferably anti-rabbit IgG antibody
(f) Incubating the product of step (e) with ProteinA and/or ProteinG-MNase fusion protein (pAG-MNase),
(g) Adding of a Ca²⁺ ions-containing buffer to start MNase digestion and release of pAG-MNase-antibody-chromatin complexes,
(h) Adding of a chelator-containing buffer to stop the reaction of step (h),
(i) Pelletizing the obtained chromatin fragments and obtaining pAG-MNase-bound digested chromatin fragments from the supernatant,
(j) Extracting of DNA and RNA, respectively, from the chromatin fragments of step (i),
(k) High-throughput sequencing of DNA and RNA, respectively,
(l) Bioinformatic identification of differential peaks of sequencing reads in samples prepared using the catalytically inactive Cas protein (dCas) or catalytically active Cas proteins (Cas)

In an alternative embodiment, the present invention concerns a method to validate CRISPR-Cas targeting comprising the following steps:
(a) Expressing a catalytically inactive Cas protein (dCas) or catalytically active Cas protein containing a protein tag and a sgRNA in target cells,
(b) Optionally hypotonic lysis of the cells of step (a) to release nuclei,
(c) Immobilizing whole cells of step (a) or nuclei of step (b) with magnetic beads, preferably Concanvalin A beads
(d) Incubating the product of step (c) with an IgG antibody against the tag of the protein tag of step (a), preferably a rabbit IgG antibody,
(e) Optionally incubating the product of step (d) with a secondary IgG antibody, preferably an anti-rabbit IgG antibody,
(f) Incubating the product of step (e) with ProteinA-MNase (pAG-MNase),
(g) Adding of a Ca²⁺ ions-containing buffer to start MNase digestion and release of pAG-MNase-antibody-chromatin complexes,
(h) Adding of a chelator-containing buffer to stop the reaction of step (g),
(i) Pelletizing the obtained oligonucleosome and obtaining pAG-MNase-bound digested chromatin fragments from the supernatant,
(j) Extracting of DNA and RNA, respectively, from the chromatin fragments of step (i),
(k) High-throughput sequencing of DNA and RNA, respectively,
(m) Bioinformatic identification of differential peaks of sequencing reads in samples prepared using the catalytically inactive Cas protein (dCas) or catalytically active Cas proteins (Cas)

In an alternative embodiment, the present invention concerns a method to validate CRISPR-Cas targeting comprising the following steps:
(a) Expressing a catalytically inactive Cas protein (dCas) or catalytically active Cas proteins (Cas) and a single or several sgRNA in target cells,
(b) Optionally hypotonic lysis of the cells of step (a) to release nuclei,
(c) Immobilizing whole cells of step (a) or nuclei of step (b) with magnetic beads, preferably Concanvalin A beads
(d) Incubating the product of step (c) with an IgG antibody against the tag of the protein of step (a), preferably a rabbit IgG antibody,
(e) Incubating the product of step (d) with a secondary IgG antibody, preferably an anti-rabbit IgG antibody
(f) Incubating the product of step (e) with a transposome comprising a protein A and/or protein G hyperactive Tn5 fusion protein (pAG-Tn5) loaded with DNA primers duplexes for high-throughput sequencing,
(g) Adding of a Ca²⁺ ions-containing buffer to start tagmentation and release of pAG-Tn5-chromatin complexes,
(h) Adding of a chelator-containing buffer to stop the reaction of step (g),
(i) Pelletizing the obtained oligonucleosome and obtaining pAG-Tn5 bound digested chromatin fragments from the supernatant,
(j) Extracting of DNA from the chromatin fragments of step (i),
(k) High-throughput sequencing of DNA.
(l) Bioinformatic identification of differential peaks of sequencing reads in samples prepared using the catalytically inactive Cas protein (dCas) or catalytically active Cas proteins (Cas)

In an alternative embodiment, the present invention concerns a method to validate CRISPR-Cas targeting comprising the following steps:
(a) Expressing a catalytically inactive Cas protein (dCas) or catalytically active Cas proteins (Cas), a single or several sgRNA, and Trex2 in target cells,
(b) Optionally hypotonic lysis of the cells of step (a) to release nuclei,
(c) Immobilizing whole cells of step (a) or nuclei of step (b) with magnetic beads, preferably Concanvalin A beads,
(d) Incubating the product of step (c) with an anti-Cas IgG antibody, preferably a rabbit anti-Cas IgG antibody
(e) Incubating the product of step (d) with a secondary IgG antibody, preferably anti-rabbit IgG antibody;
(f) Incubating the product of step (d) with a transposome comprising a protein A and/or protein G hyperactive Tn5 fusion protein (pAG-Tn5) loaded with DNA primers duplexes for high-throughput sequencing,
(g) Adding of a Ca²⁺ ions-containing buffer to start tagmentation and release of pAG-Tn5-chromatin complexes,
(h) Adding of a chelator-containing buffer to stop the reaction of step (g),
(i) Pelletizing the obtained oligonucleosome and obtaining pAG-Tn5 bound digested chromatin fragments from the supernatant,
(j) Extracting of DNA from the chromatin fragments of step (i),
(k) High-throughput sequencing of DNA,
(l) Bioinformatic identification of differential peaks of sequencing reads in samples prepared using the catalytically inactive Cas protein (dCas) or catalytically active Cas proteins (Cas)

In an alternative embodiment, the present invention concerns a method to validate CRISPR-Cas targeting comprising the following steps:
(a) Expressing a catalytically inactive Cas protein (dCas) or catalytically active Cas proteins (Cas) containing a protein tag and a single or several sgRNA in target cells,
(b) Optionally hypotonic lysis of the cells of step (a) to release nuclei,
(c) Immobilizing whole cells of step (a) or nuclei of step (b) with magnetic beads, preferably Concanvalin A beads
(d) Incubating the product of step (c) with an IgG antibody against the tag of the protein of step (a), preferably a rabbit IgG antibody
(e) Incubating the product of step (d) with a secondary IgG antibody, preferably anti-rabbit antibody
(f) Incubating the product of step (d) with a transposome comprising a protein A and/or protein G hyperactive Tn5 fusion protein loaded with DNA primers duplexes for high-throughput sequencing.
(g) Adding of a Ca²⁺ ions-containing buffer to start tagmentation and release of pAG-Tn5-chromatin complexes,
(h) Adding of a chelator-containing buffer to stop the reaction of step (f),
(i) Pelletizing the obtained oligonucleosome and obtaining pAG-MNase-bound digested chromatin fragments from the supernatant,
(j) Extracting of DNA from the chromatin fragments of step (h),
(l) High-throughput sequencing of DNA,
(l) Bioinformatic identification of differential peaks of sequencing reads in samples prepared using the catalytically inactive Cas protein (dCas) or catalytically active Cas proteins (Cas)

### CUT&RUN Protocol

MNase digestion and cleavage product release can be achieved under standard CUT&RUN conditions or high Ca²⁺/low salt conditions. The latter options is particularly preferable for smaller sample sizes, as it can potentially reduce background signals.

### High Ca²⁺/low salt chromatin cleavage

This protocol option corresponds to a more recent improvement of the CUT&RUN protocol (Meers, M. P., Bryson, T. D., Henikoff, J. G. & Henikoff, S. Improved CUT&RUN chromatin profiling tools. Elife 8, e46314 (2019); PMID 31232687). It is intended to reduce background due to DNA overdigestion by free pAG-MNase-antibody-chromatin complexes.

The protocol takes advantage of the fact that nucleosomes aggregate in the presence of high concentrations of divalent cations (e.g. 5-20 mM, preferably 7-15 mM, more preferably about 10 mM Ca²⁺) and at low salt concentrations (e.g. 10-50 mM, preferably about 25 mM) to reduce release of the pAG-MNase-antibody-chromatin cleavage products. Subsequently to the digestion of the samples in high Ca²⁺/low salt conditions, cleavage products are released in a high salt buffer containing a chelator (e.g. ethyleneglycol-bis(β-aminoethyl) -N,N,N',N'-tetraacetic acid (EGTA)) to prevent further DNA cleavage.

As mentioned above, premature release of cleavage product particles during the digestion step can cause MNase off-site cleavage and thus increased background signal. This is particularly relevant when cleaving chromatin under abundant targets for longer digestions times causes increased background. Longer retention of the cleavage product particles within the nucleus could also improve CUT&RUN with lower cell numbers.

### Accurate quantitation with Spike-in DNA or carry-over E.coli DNA

Heterologous spike-in DNA in the Stop Buffer allows the comparison of DNA yields between different samples. The total number of spike-in DNA sequencing reads serve as normalization factor and are inversely proportional to the total number of sample DNA sequencing reads (Skene, P. J. & Henikoff, S. An efficient targeted nuclease strategy for high-resolution mapping of DNA binding sites. Elife 6, 1-35 (2017); PMID 28079019). Spike-in DNA should be fragmented down to an average length of approximately 100-300 bp, preferably about 200 bp. The amount of spike-in DNA can be adjusted based on the number of cells collected for each sample: use 50-200 pg/mL, preferably about 100 pg/mL for 10⁴-10⁶ cells and 0.5-5 pg/mL, preferably about 2 pg/mL for 10²-10⁴ cells.

Alternatively, *E. coli* carry-over DNA from the purification of the pAG-MNase fusion protein has been shown to be a viable calibration standard (Meers, M. P., Bryson, T. D., Henikoff, J. G. & Henikoff, S. Improved CUT&RUN chromatin profiling tools. Elife 8, e46314 (2019); PMID 31232687). As it is introduced at step 46 in the following Example 1, it is digested by the MNase and released at the same time as the sample chromatin DNA. In this case, no heterologous spike-in DNA needs to be added to the Stop Buffer.

The invention will be further described by the accompanying figures which show:
- Fig. 1:: ABC-seq (CUT& RUN) for CRISPR/Cas binding sites
- Fig. 2:: ABC-seq (CUT& RUN) for CRISPR/Cas cleavage sites
- Fig. 3:: ABC-seq (CUT& TAG) for CRISPR/Cas binding sites
- Fig. 4:: ABC-seq (CUT&TAG) for CRISPR/Cas cleavage sites

The invention is further described by the following Examiner, which, however, are not construed as a limitation of the scope of the invention.

### Example 1: ABC-seq (CUT&RUN) using high Ca²⁺/low salt chromatin cleavage

### Buffer preparation

### • Wash buffer (100 mL)

| Com ponent | Volume | Final concentration |
|---|---|---|
| ddH₂O | 94 mL | - |
| 1 M HEPES pH 7.5 | 2 mL | 20 mM |
| 5 M NaCl | 3 mL | 150 mM |
| 2 M Spermidine | 25 µL | 0.5 mM |

∘ Store Wash Buffer without protease inhibitors for up to one week at 4 °C.
∘ Add protease inhibitor fresh before use:

| | | |
|---|---|---|
| EDTA-free Protease Inhibitor Cocktail 100x | 1 mL | 1x |

### • Binding Buffer (40 mL)

| Component | Volume | Final concentration |
|---|---|---|
| ddH₂O | 39 ml | - |
| 1 M HEPES pH 7.5 | 800 µl | 20 mM |
| 1 M KCI | 400 µl | 10 mM |
| 1 M CaCl₂ | 40 µl | 1 mM |
| 1 M MnCl₂ | 40 µl | 1 mM |

∘ Store Binding Buffer for up to six months at 4 °C.

### • Digitonin Wash Buffer (70 mL)

| Com ponent | Volume | Final concentration |
|---|---|---|
| 5% Digitonin | 350-1400 µL | 0.025%-0.1% |
| Wash Buffer | 69 mL | - |

∘ Store Digitonin Wash Buffer for up to one day at 4 °C.
∘ Recommended Digitonin concentration ranges from 0.025% to 0.1%.

### • Antibody Buffer (2 mL)

| Com ponent | Volume | Final concentration |
|---|---|---|
| 0.5 M EDTA | 8 µL | 2 mM |
| Digitonin Wash Buffer | 2 mL | - |

∘ Store Antibody Buffer for up to one day at 4 °C until use.

### • 100 mM CaCl₂ (2 mL)

| Com ponent | Volume | Final concentration |
|---|---|---|
| CaCl₂ | 200 µL | 100 mM |
| ddH₂O | 1,800 µl | - |

### • Low Salt Rinse Buffer (35 mL)

| Com ponent | Volume | Final concentration |
|---|---|---|
| ddH₂O | 34 mL | - |
| 1 M HEPES pH 7.5 | 700 µL | 20 mM |
| 2 M Spermidine | 8.75 µL | 0.5 mM |
| 5% Digitonin | 350 µL | 0.05% |

∘ Store Low Salt Rinse Buffer for up to one week at 4 °C until use.

### • Low Salt Incubation Buffer (4 mL)

| Com ponent | Volume | Final concentration |
|---|---|---|
| ddH₂O | 3906 µL | - |
| 1 M HEPES pH 7.5 | 14 µL | 3.5 mM |
| 1 M CaCl₂ | 40 µL | 10 mM |
| 5% Digitonin | 40 µL | 0.05% |

∘ Store Low Salt Incubation Buffer for up to one week at 4 °C until use.

### • Low Salt Stop Buffer (4 mL)

| Com ponent | Volume | Final concentration |
|---|---|---|
| ddH₂O | 3400 µL | - |
| 5 M NaCl | 136 µL | 170 mM |
| 0.2 M EGTA | 400 µL | 20 mM |

∘ Store Low Salt Stop Buffer at 4 °C until use.
∘ Add fresh before use

| Com ponent | Volume | Final concentration |
|---|---|---|
| 5% Digitonin | 40 µL | 0.05% |
| RNase A (10 mg/mL) | 20 µL | 50 µg/mL L |
| Glycogen (20 mg/mL) | 5 µL | 25 µg/mL L |
| heterologous spike-in DNA | | 100 pg/ml |

### ABC-seq (CUT&RUN) protocol using a mouse monoclonal anti- SpCas9 antibody

### I. Expression of a catalytically inactive Streptococcus pyogenes Cas protein (dSpCas9) and a catalytically active SpCas9 in target cells

1. Culture human K-562 cells.
2. Transfect cells using e.g. Lipofectamine 3000 (ThermoFisher Scientific, Waltham/MA, USA) according to the manufacturer's instructions with the following expression vectors
   - a dCas9 plasmid containing a catalytically inactive dSpCas9 construct and one or several sgRNA targeting (a) known genomic site(s) (e.g. pRP[Exp]-U6>gRNA#1-U6>gRNA#2-U6>gRNA#3-CBh>3xFlag/SV40 NLS/dCas9/NLS:T2A:EGFP/Neo; antibodies-online, Aachen, Germany) and a plasmid expressing Trex2 or
   - a Cas9 plasmid containing a catalytically active SpCas9 construct and one or several sgRNA targeting (a) known genomic site(s) (e.g. pRP[Exp]-U6>gRNA#1-U6>gRNA#2-U6>gRNA#3-CBh>3xFlag/SV40 NLS/Cas9/NLS:T2A:EGFP/Neo; antibodies-online, Aachen, Germany) and a plasmid expressing Trex2 or
   - a dCas9 plasmid containing a catalytically inactive dSpCas9 construct and a scrambled sgRNA (pRP[Exp]-U6>Scramble[gRNA#1]-CBh>3xFlag/SV40 NLS/dCas9/NLS:T2A:EGFP/Neo; antibodies-online, Aachen, Germany) and a plasmid expressing Trex2 or
   - a mock transfection not containing any plasmid DNA.

### II. Cell harvest

3. Harvest 100.000 cells for each sample at room temperature. Keep cells for each sample in separate tubes.
4. Centrifuge cell solution 3 min at 600 x g at room temperature.
5. Remove the liquid carefully.
6. Gently resuspend cells in 1 mL Wash Buffer by pipetting and transfer cell solution to a 1.5 mL microcentrifuge tube.
7. Centrifuge cell solution 3 min at 600 x g at room temperature and discard the supernatant.
8. Repeat steps 4-5 thrice for a total of four washes.
9. Resuspend cell pellet for each sample in 1 mL Wash Buffer by gently pipetting.

### III. Concanavalin A beads preparation

10. Prepare one 1.5 mL microcentrifuge tube for each sample.
11. Gently resuspend the CUT&RUN Concanavalin A Beads (antibodies-online, Aachen, Germany, #ABIN6952467).
12. Pipette 10 µL Concanavalin A Beads slurry for each sample into the 1.5 mL microcentrifuge tubes.
13. Place the tubes on a magnet stand until the fluid is clear. Remove the liquid carefully.
14. Remove the microcentrifuge tube from the magnetic stand.
15. Pipette 1 mL Binding Buffer into each tube and resuspend Concanavalin A Beads by gentle pipetting.
16. Spin down the liquid from the lid with a quick pulse in a table-top centrifuge (max 100 x g).
17. Place the tubes on a magnet stand until the fluid is clear. Remove the liquid carefully.
18. Remove the microcentrifuge tube from the magnetic stand.
19. Repeat steps 13-16 twice for a total of three washes.
20. Gently resuspend the Concanavalin A Beads in a volume of Binding Buffer corresponding to the original volume of bead slurry, i.e. 10 µL per sample.

### IV. Cell immobilization on Concanavalin A beads

21. Carefully vortex the cell suspension from step 9 and add 10 µL of the Concanavalin A Beads in Binding Buffer prepared in section II to each sample.
22. Close tubes tightly and rotate for 5-10 min at room temperature.

### V. Cell permeabilization and antibody binding

23. Place the microcentrifuge tubes on a magnetic stand until the fluid is clear. Remove the liquid carefully.
24. Remove the microcentrifuge tubes from the magnetic stand.
25. Place each tube at a low angle on the vortex mixer set to a low speed (approximately 1,100 rpm) and add 100 µL Antibody Buffer containing digitonin.
26. Gently vortex the microcentrifuge tubes until the beads are resuspended.
27. Add 1 µL antibody corresponding to a 1:100 dilution to each tube:
   - positive control rabbit anti-H3K4me3 antibody (antibodies-online, Aachen, Germany, #ABIN3023255)
   - monoclonal mouse anti-CRISPR-Cas9 antibody (antibodies-online, Aachen, Germany, #ABIN4880057)
   - polyclonal rabbit anti-CRISPR-Cas9 antibody (antibodies-online, Aachen, Germany, #ABIN5596701)
   - negative control polyclonal guinea pig anti-rabbit IgG antibody (antibodies-online, Aachen, Germany, #ABIN101961)
28. Rotate the microcentrifuge tubes overnight at 4 °C.
29. Spin down the liquid and place the tubes on a magnet stand until the fluid is clear. Remove the liquid carefully.
30. Remove the microcentrifuge tubes from the magnetic stand.
31. Resuspend with 1 ml Digitonin Wash Buffer and mix by inversion. If clumping occurs, gently remove the clumps with a 1 ml pipette tip.
32. Repeat steps 29-31 once for a total of two washes.

### VI. Secondary antibody binding

33. Place the tubes containing the samples incubated with the anti-Cas9 antibodies on a magnet stand until the fluid is clear. Remove the liquid carefully.
34. Remove the microcentrifuge tubes from the magnetic stand.
35. Vortex the sample at low speed (approx. 1,100 rpm) and add 100 µL Digitonin Wash Buffer per sample along the side of the tube.
36. Tap to remove the remaining beads from the tube side.
37. Add 1 µL of the following antibodies to a 1:100 dilution into the respective microcentrifuge tube:
   - Polyclonal rabbit anti-mouse IgG antibody (antibodies-online, Aachen, Germany, #ABIN101785) to the tube incubated with the monoclonal mouse anti-CRISPR-Cas9 antibody (antibodies-online, Aachen, Germany, #ABIN4880057)
   - polyclonal guinea pig anti-rabbit IgG antibody (antibodies-online, Aachen, Germany, #ABIN101961) to the tube incubated with the polyclonal rabbit anti-CRISPR-Cas9 antibody (antibodies-online, Aachen, Germany, #ABIN5596701)
38. Rotate the microcentrifuge tubes for 1 h at 4 °C.
39. Spin down the liquid and place the tubes on a magnet stand until the fluid is clear. Remove the liquid carefully.
40. Remove the microcentrifuge tubes from the magnetic stand.
41. Resuspend with 1 mL Digitonin Wash Buffer and mix by inversion. If clumping occurs, gently remove the clumps with a 1 ml pipette tip.
42. Repeat steps 39-41 once for a total of two washes.

### VII. pAG-MNase Binding

43. Place all tubes (positive and negative control from step 32, samples from step 42) on a magnet stand until the fluid is clear. Remove the liquid carefully.
44. Remove the microcentrifuge tubes from the magnetic stand.
45. Place each tube at a low angle on the vortex mixer set to a low speed (approximately 1,100 rpm) and add 50 µL Digitonin Wash Buffer per sample alongside of the tube.
46. Add 2.5 µL pAG-MNase (antibodies-online, Aachen, Germany, #ABIN6950951)
47. Rotate the microcentrifuge tubes for 1 h at 4 °C.
48. Spin down the liquid and place the tubes on a magnet stand until the fluid is clear. Remove the liquid carefully.
49. Remove the microcentrifuge tubes from the magnetic stand.
50. Resuspend with 1 ml Digitonin Wash Buffer and mix by inversion. If clumping occurs, gently remove the clumps with a 1 ml pipette tip.
51. Repeat steps 48-50 once for a total of two washes.

### VIII. High Ca²⁺/low salt MNase chromatin cleavage and release of pAG-MNase-antibody-chromatin complexes

52. Spin down the liquid from the lid with a quick pulse in a table-top centrifuge (max 100 x g).
53. Place the tubes on a magnet stand until the fluid is clear. Remove the liquid carefully.
54. Resuspend with 1 mL Low Salt Rinse Buffer and mix by inversion. If clumping occurs, gently remove the clumps with a 1 mL pipette tip.
55. Spin down the liquid from the lid with a quick pulse in a table-top centrifuge (max 100 x g)
56. Place the tubes on a magnet stand until the fluid is clear. Remove the liquid carefully.
57. Repeat steps 54-56 once for a total of two washes.
58. Place each tube at a low angle on the vortex mixer set to a low speed (approximately 1,100 rpm) and add 200 µL ice cold Low Salt Incubation Buffer per sample along the side of the tube.
59. Incubate tubes at 0°C for 30 min.
60. Place the tubes on a cold magnet stand until the fluid is clear. Remove the liquid carefully.
61. Remove the microcentrifuge tubes from the magnetic stand.
62. Resuspend with 200 µL Low Salt Stop Buffer and mix by gentle vortexing.
63. Incubate tubes at 37 °C for 30 min.
64. Place the tubes on a magnet stand until the fluid is clear.
65. Transfer the supernatant containing the pAG-MNase-bound digested chromatin fragments to fresh 1.5 mL microcentrifuge tubes.

### IX. DNA extraction

66. Add 2 µL 10% SDS to a final concentration of 0.1%, 5 µL Proteinase K (10 mg/ml) to a final concentration of 2.5 mg/mL, and 1 µL RNase (10 mg/mL) to a final concentration of 50 µg/mL to each supernatant from step 65.
67. Gently vortex tubes at a low speed of approximately 1,100 rpm.
68. Incubate tubes at 50 °C for 1 h.
69. Add 200 µL PCI to tube.
70. Shake tubes thoroughly at high speed until the liquid appears milky.
71. Centrifuge tubes in a tabletop centrifuge at 16,000 x g at 4 °C for 5 min.
72. Carefully transfer the upper aqueous phase to a fresh 1.5 mL microcentrifuge tube containing 200 µL chloroform:isoamyl alcohol 24:1.
73. Vortex tubes thoroughly at high speed until the liquid appears milky.
74. Centrifuge tubes in a tabletop centrifuge at 16,000 x g at 4 °C for 5 min.
75. Carefully transfer to upper aqueous phase to a fresh 1.5 mL microcentrifuge tube containing 2 µL glycogen (diluted 1:10 to 2 mg/mL from the 20 mg/mL stock solution).
76. Add 20 µL 3 M NaOAc pH 5.2 and 500 µL 100% ethanol.
77. Place tubes overnight at -20 °C.
78. Centrifuge tubes in a tabletop centrifuge at 16,000 x g at 4 °C for 20 min.
79. Remove the liquid carefully with a pipette.
80. Add 1 ml 70% ethanol.
81. Centrifuge tubes in a tabletop centrifuge at 16,000 x g at 4 °C for 5 min.
82. Remove the liquid carefully with a pipette.
83. Dry the pellet in a SpeedVac.
84. Dissolve the pellet in 30 µL 1 mM Tris-HCI, 0.1 mM EDTA.

### X. Sample quality control

85. Determine DNA concentration using a Quantus fluorometer.
86. Check size distribution of the DNA fragments on a Tapestation

### XI. Sequencing library preparation and sequencing

87. Prepare the CUT&RUN products sequencing libraries according to the workflow described in PMID 31500663 using an NEBNext Ultra II DNA Library Prep Kit for Illumina.
88. Pool sequences with different indices and perform 36 bp paired-end sequencing at a sequencing depth of 0.12x to 0.15x coverage of the human genome.

### XII. Peak calling and comparative analysis of SpCas and dSpCas data sets

89. Quality control of the sequencing reads (e.g. FastQC).
90. Trim raw sequencing reads to avoid adapter contamination in short sequencing reads.
91. Sequencing read alignment optimized for short sequencing reads (using e.g. Bowtie2)
92. Peak calling of aligned sequencing reads optimized for short sequencing reads (e.g. SEACR, MACS2). The mock transfected cells treated with the unspecific negative control antibody serves to establish a baseline.
93. Call differential peaks to identify SpCas9 binding sites and cleavage sites (e.g. Diffbind, HOMER):
   - Peaks appearing in datasets for the catalytically inactive dSpCas9 and the active SpCas9 correspond to binding sites.
   - Peaks appearing only in datasets for the catalytically inactive dSpCas9 but not SpCas9 correspond to cleavage sites.
   - Peaks appearing in datasets for the catalytically inactive dSpCas9 with the specific gRNA(s) and for the catalytically inactive dSpCas9 with the scramble gRNA correspond to sequence-independent SpCas9 binding sites.

### Example 2: ABC-seq (CUT&Tag)

### • Binding Buffer (5 mL)

| Component | Volume | Final concentration |
|---|---|---|
| ddH₂O | 4.85 mL | |
| 1 M HEPES pH 7.5 | 100 µL | 20 mM |
| 1 M KCI | 50 µL | 10 mM |
| 1 M CaCl₂ | 5 µL | 1 mM |
| 2.5 M MnCl₂ | 2 µl | 1 mM |

∘ Store Binding Buffer for up to six months at 4 °C.

### • Wash buffer (70 ml)

| Com ponent | Volume | Final concentration |
|---|---|---|
| ddH₂O | 66 mL | - |
| 1 M HEPES pH 7.5 | 1.4 mL | 20 mM |
| 5 M NaCl | 2.1 mL | 150 mM |

∘ Add protease inhibitor fresh before use

| | | |
|---|---|---|
| 2 M Spermidine | 15.5 µL | 0.5 mM |
| EDTA-free Protease Inhibitor Cocktail 100x | 700 µL | 1x |

∘ Once Spermidine and Protease Inhibitor have been added, store the Wash Buffer at 4°C and use up within two days or store at -20°C.

### • Digitonin Wash Buffer (45 mL)

| Com ponent | Volume | Final concentration |
|---|---|---|
| 5% Digitonin | 225 µL | 0.025% |
| Wash Buffer | 45 mL | - |

∘ Store Digitonin Wash Buffer for up to one day at 4 °C.
∘ Recommended Digitonin concentration ranges from 0.025% to 0.1%.

### • Antibody Buffer (1.5 mL)

| Com ponent | Volume | Final concentration |
|---|---|---|
| 0.5 M EDTA | 6 µL | 2 mM |
| 10% BSA | 15 µL | 0.1% |
| Digitonin Wash Buffer | 1.5 mL | - |

∘ Store Antibody Buffer for up to one day at 4 °C until use.

### • Dig-300 Buffer (48 mL)

| Com ponent | Volume | Final concentration |
|---|---|---|
| ddH₂O | 154 mL | - |
| 1 M HEPES pH 7.5 | 960 µL | 20 mM |
| 5 M NaCl | 2.88 ml | 300 mM |
| 2 M Spermidine | 12 µL | 0.5 mM |

∘ Store Dig-300 Buffer without protease inhibitors and Digitonin for up to one week at 4 °C.
∘ Add protease inhibitor and Digitonin fresh before use, e.g.

| | | |
|---|---|---|
| Protease Inhibitor Cocktail (EDTA-free) 100x | 480 µL | 1x |
| 5% Digitonin | 96 µL | 0.01% |

### • Tagmentation Buffer (4.2 mL)

| Com ponent | Volume | Final concentration |
|---|---|---|
| Dig-300 Buffer | 4.2 mL | - |
| 1 M MgCl₂ | 42 µl | 10 mM |

∘ Prepare Tagmentation Buffer fresh before use.

### • Oligonucleotides (for lllumina)

| Oliognucleotide | Nucleotide sequence | Concentrati on |
|---|---|---|
| Mosaic end - adpater A (ME-A) | TCGTCGGCAGCGTCAGATGTGTATA AGAGACAG | 100□µM |
| Mosaid end - adapter B (ME-B) | GTCTCGTGGGCTCGGAGATGTGTAT AAGAGACAG | 100□µM |
| Mosaic end - reverse (ME-rev) | Phos-CTGTCTCTTATACACATCT | 100□µM |
| Universal i5 primer | AATGATACGGCGACCACCGAGATCT ACACTCGTCGGCAGCGTCAGATGTG | 10 µM |
| Uniquely barcoded i7 primer | CAAGCAGAAGACGGCATACGAGAT -8nt barcode- GTCTCGTGGGCTCGGAGATGT | 10 µM |

### ABC-seq (CUT&Tag) protocol using a mouse monoclonal anti- SpCas9 antibody

### I. pAG-Tn5 adapter complex assembly

1. Prepare one 0.5 mL PCR tube for each of the ME-A/ME-rev and ME-B/ME-rev oligonucleotide duplexes.
2. Combine 10 µL 100 µM ME-A or ME-B oligonucleotide with 10 µM ME-rev oligonucleotide in the respective tubes.
3. Place tubes in a heating block at 95 °C for 5 min.
4. Keep tubes in the heating block and remove the heating block from the dry block incubator. Let the heating block cool down on the bench top to RT.
5. Mix 8µl of each of the preannealed ME-A/ME-rev and ME-B/ME-rev oligonucleotide duplexes at 100 µM with 100 µL of 5.5 µM pAG-Tn5 fusion protein.
6. Incubate the mixture on a rotating platform for 1 h at RT and then store at - 20 °C.

### II. Cell harvest

7. Harvest a cell number (cells as obtained in Example 1, I.) corresponding to up to 100,000 mammalian cells for the positive control, negative control, and each sample plus one at room temperature; e.g. 1.3x10⁶ cells for 10 samples and the two controls.
8. Centrifuge cell solution 3 min at 600 x g at room temperature.
9. Remove the liquid carefully.
10. Resuspend cells in a volume of Wash Buffer corresponding to the volume of the cell solution or at most 10 mL by pipetting.
11. Centrifuge cell solution 3 min at 600 x g at room temperature.
12. Remove the liquid carefully.
13. Resuspend cells in 1.2 mL Wash Buffer by pipetting and transfer cell solution to a 1.5 mL microcentrifuge tube.
14. Centrifuge cell solution 3 min at 600 x g at room temperature and discard the supernatant.
15. Resuspend cell pellet in 100 µL Wash Buffer for each sample plus one by gently pipetting; e.g. 1.3 mL for 10 samples and the two controls.

### III. Concanavalin A beads preparation

16. Gently resuspend the CUT&RUN Concanavalin A Beads (purple dot).
17. Pipette a volume of CUT&RUN Concanavalin A Beads slurry corresponding to 10 µL for the positive control, negative control, and each sample plus one into a 1.5 mL microcentrifuge tube containing 1.2 mL Binding Buffer; e.g. 130 µL CUT&RUN Concanavalin A Beads slurry for 10 samples and the two controls.
18. Place the tube on a magnet stand until the fluid is clear.
19. Remove the liquid carefully and remove the microcentrifuge tubes from the magnetic stand.
20. Resuspend CUT&RUN Concanavalin A Beads in 1 mL Binding Buffer by gentle pipetting.
21. Spin down the liquid from the lid with a quick pulse in a table-top centrifuge (max 100 x g).
22. Place the tubes on a magnet stand until the fluid is clear.
23. Remove the liquid carefully and remove the microcentrifuge tubes from the magnetic stand.
24. Repeat steps 20-23 once for a total of two washes.
25. Gently resuspend the CUT&RUN Concanavalin A Beads in a volume of Binding Buffer corresponding to the original volume of bead slurry, i.e. 10 µL per sample and control; e.g. 130 µL CUT&RUN Binding Buffer for 10 samples and the two controls.

### IV. Cell immobilization - binding to Concanavalin A beads

26. Carefully vortex the cell suspension from step 15 and add the CUT&RUN Concanavalin A Beads in Binding Buffer from step 25.
27. Close tube tightly and rotate for 5-10 min at room temperature.

### V. Cell permeabilization and primary antibody binding

28. Prepare one 1.5 mL microcentrifuge tube for each sample and the two controls.
29. Place the microcentrifuge tube from step 27 on a magnetic stand until the fluid is clear.
30. Carefully remove the liquid from the cells immobilized on the CUT&RUN Concanavalin A Beads.
31. Remove the microcentrifuge tubes from the magnetic stand.
32. Gently resuspend the beads in a volume of ice cold Antibody Buffer containing digitonin corresponding to 100 µL per sample and control; e.g. 1.3 mL Antibody Buffer for 10 samples and the two controls.
33. Pipette 100 µL aliquots of the CUT&RUN Concanavalin A Beads in Antibody Buffer into the 1.5 mL microcentrifuge tubes prepared in step 28.
34. For the positive control, add 5 µL CUT&Tag rabbit anti-H3K4me3 IgG Positive Control (turquois dot) corresponding to a 1:20 dilution to the corresponding tube.
35. For the negative control, do not add anything else to the corresponding tube.
36. For the remaining samples, 1 µL anti-Cas9 primary rabbit antibody (Antibodies-online, Aachen, Germany, #ABIN2670026) corresponding to a 1:100 dilution.
37. Rotate the microcentrifuge tubes for 2 h at room temperature or overnight at 4 °C.
38. Quickly spin down the liquid and place the tubes on a magnet stand until the fluid is clear.
39. Remove the liquid carefully and remove the microcentrifuge tubes from the magnetic stand.

### VI. Secondary antibody binding

40. Add 100 µL Digitonin Wash Buffer per tube along the side of the microcentrifuge tube and vortex at low speed (approximately 1,100 rpm).
41. Tap to remove the remaining beads from the tube side.
42. Add 5 µL CUT&Tag Secondary Antibody corresponding to a 1:20 dilution.
43. Rotate the microcentrifuge tubes for 1 h at room temperature.
44. Spin down the liquid and place the tubes on a magnet stand until the fluid is clear.
45. Remove the liquid carefully and remove the microcentrifuge tubes from the magnetic stand.
46. Resuspend with 1 mL Digitonin Wash Buffer and mix by inversion. If clumping occurs, gently remove the clumps with a 1 ml pipette tip.
47. Repeat steps 44-46 twice for a total of three washes.

### VII. pAG-Tn5 adapter complex binding

48. Dilute the pAG-Tn5 adapter complex ABIN from step 6 1:250 in a volume of Dig-300 Buffer corresponding to 100 µL per sample; e.g. 5.2 µL pAG-Tn5 adapter complex in 1.3 mL for for 10 samples and the two controls.
49. Place the tubes from step 47 on a magnet stand until the fluid is clear.
50. Remove the liquid carefully and remove the microcentrifuge tubes from the magnetic stand.
51. Place each tube at a low angle on the vortex mixer set to a low speed (approximately 1,100 rpm) and add 100 µL pAG-Tn5 adapter complex in Dig-300 Buffer from step 48 along the side of the tube.
52. Rotate the microcentrifuge tubes for 1 h at room temperature.
53. Spin down the liquid and place the tubes on a magnet stand until the fluid is clear.
54. Remove the liquid carefully and remove the microcentrifuge tubes from the magnetic stand.
55. Resuspend with 1 ml Dig-300 Buffer and mix by inversion. If clumping occurs, gently remove the clumps with a 1 ml pipette tip.
56. Repeat steps 53-55 twice for a total of three washes.

### VIII. Tagmentation

57. Spin down the liquid from the lid with a quick pulse in a table-top centrifuge (max 100 x g).
58. Place the tubes on a magnet stand until the fluid is clear.
59. Remove the liquid carefully and remove the microcentrifuge tubes from the magnetic stand.
60. Place each tube at a low angle on the vortex mixer set to a low speed (approximately 1,100 rpm) and add 300 µL Tagmentation Buffer along the side of the tube.
61. Spin down the liquid from the lid with a quick pulse in a table-top centrifuge (max 100 x g)
62. Rotate the microcentrifuge tubes for 1 h at 37 °C.

### IX. DNA extraction

63. Add 10 µL 0.5 M EDTA to a final concentration of 16 mM, 3 µL 10% SDS to a final concentration of 0.1%, and 7.5 µL Proteinase K (10 mg/mL) to a final concentration of 0.25 mg/mL to each reaction.
64. Vortex tubes thoroughly at a high speed.
65. Incubate tubes at 50 °C for 1 h or at 37 °C ON.
66. Without separating the liquid supernatant and the beads add 300 µL PCI to each tube.
67. Vortex tubes thoroughly at high speed until the liquid appears milky.
68. Transfer liquid to a 1.5 mL phase-lock tube.
69. Add 300 µL chloroform and mix by inversion.
70. Centrifuge tubes in a tabletop centrifuge at 16,000 x g at room temperature for 3 min.
71. Using a pipette, transfer the aqueous layer to a new tube containing 750 µL 100% ethanol.
72. Transfer tubes to a cold tabletop centrifuge and centrifuge at 16,000 x g at 4 °C for 15 min.
73. Carefully pour off the liquid and remove the remaining liquid with a pipette.
74. Add 1 mL 100% ethanol.
75. Carefully pour off the liquid, remove the remaining liquid with a pipette, and air dry the tubes.
76. Dissolve the pellet in 23 µL TE containing RNase A diluted 1:400 to 25 ng/mL.
77. Incubate tubes at 37 °C for 10 min.

### X. PCR amplification and Clean-Up

78. Transfer 21 µl into a 0.5 mL PCR tube.
79. Add 2 µL Universal i5 Primer at 10 µM and 2 µL i7 Primer at 10 µM with a unique barcode for each sample.
80. Add 25 µL PCR master mix of a high fidelity polymerase (e.g. NEBNext Ultra II Q5 Master Mix, Roche KAPA Library Amplification Kit).
81. Mix tubes thoroughly by vortexing.
82. Spin down the liquid from the lid with a quick pulse (max 100 x g).
83. PCR program:

| | | |
|---|---|---|
| step 1 | 58 °C | 5 min |
| step 2 | 72 °C | 30 sec |
| step 3 | 98 °C | 30 sec |
| step 4 | 98 °C | 10 sec |
| step 5 | 60 °C | 10 sec |
| step 6 | goto step 4 | 14 times |
| step 7 | 72 °C | 1min |
| | 4 °C | hold |

84. Transfer the PCR reactions to 1.5 mL microcentrifuge tubes.
85. Add 1.3x volumes (65 µL for a 50 µL PCR mix) SPRI bead slurry and mix by pipetting.
86. Place the tubes on a magnet stand until the fluid is clear.
87. Remove the liquid carefully with a pipette and keep the microcentrifuge tubes on the magnetic stand.
88. Add 200 µL 80% ethanol.
89. Remove the liquid carefully with a pipette and remove the microcentrifuge tubes from the magnetic stand.
90. Immediately add 25 µL 10□mM Tris-HCI pH 8.0 and mix by pipetting. Elute DNA for at RT for 5 min.
91. Place the tubes on a magnet stand until the fluid is clear.
92. Transfer liquid to fresh 1.5 mL microcentrifuge tubes.

### XI. Sample quality control

93. Determine DNA concentration using a Quantus fluorometer.
94. Check size distribution of the DNA fragments on a Tapestation

### XIII. Sequencing library preparation and sequencing

95. Prepare the CUT&RUN products sequencing libraries according to the workflow described in PMID 31500663 using an NEBNext Ultra II DNA Library Prep Kit for Illumina.
96. Pool sequences with different indices and perform 36 bp paired-end sequencing at a sequencing depth of 0.12x to 0.15x coverage of the human genome.

### XIV. Peak calling and comparative analysis of SpCas and dSpCas data sets

94. Quality control of the sequencing reads (e.g. FastQC).
95. Trim raw sequencing reads to avoid adapter contamination in short sequencing reads.
96. Sequencing read alignment optimized for short sequencing reads (using e.g. Bowtie2)
97. Peak calling of aligned sequencing reads optimized for short sequencing reads (e.g. SEACR, MACS2). The mock transfected cells treated with the unspecific negative control antibody serves to establish a baseline.
98. Call differential peaks to identify SpCas9 binding sites and cleavage sites (e.g. DESeq2, Diffbind, HOMER):
   - Peaks appearing in datasets for the catalytically inactive dSpCas9 and the active SpCas9 correspond to binding sites.
   - Peaks appearing only in datasets for the catalytically inactive dSpCas9 but not SpCas9 correspond to cleavage sites.
      Peaks appearing in datasets for the catalytically inactive dSpCas9 with the specific gRNA(s) and for the catalytically inactive dSpCas9 with the scramble gRNA correspond to sequence-independent SpCas9 binding sites.

## Claims

1. A method to comprehensively capture and analyze CRISPR-Cas binding and cleavage sites comprising the following steps:
(a) Expressing a catalytically inactive Cas protein (dCas) or catalytically active Cas proteins (Cas) and a single or several sgRNA in target cells,
(b) Optionally hypotonic lysis of the cells of step (a) to release nuclei,
(c) Immobilizing whole cells of step (a) or nuclei of step (b) with magnetic beads,
(d) Incubating the product of step (c) with an anti-Cas antibody,
(e) Incubating the product of step (d) with ProteinA-ProteinG-MNase fusion protein (pAG-MNase),
(f) Adding of a Ca²⁺ ions-containing buffer to start MNase digestion and release of pAG-MNase-antibody-chromatin complexes,
(g) Adding of a chelator-containing buffer to stop the reaction of step (f),
(h) Pelletizing the obtained chromatin fragments and obtaining pAG-MNase-bound digested chromatin fragments from the supernatant,
(i) Extracting of DNA and RNA, respectively, from the chromatin fragments of step (h),
(j) High-throughput sequencing of DNA and RNA, respectively.
(k) Identification of differential peaks of sequencing reads in samples prepared using the catalytically inactive Cas protein (dCas) or catalytically active Cas proteins (Cas)

2. The method of claim 1, wherein in step (a) 3' repair exonuclease 2 (Trex2) is added.

3. A method to comprehensively capture and analyze CRISPR-Cas binding and cleavage sites independently of an sgRNA comprising the following steps:
(a) Expressing a catalytically inactive Cas protein (dCas) or catalytically active Cas protein without sgRNA,
(b) Optionally hypotonic lysis of the cells of step (a) to release nuclei,
(c) Immobilizing whole cells of step (a) or nuclei of step (b) with magnetic beads,
(d) Incubating the product of step (c) with an anti-Cas antibody,
(e) Incubating the product of step (d) with ProteinA and/or ProteinG-MNase fusion protein (pAG-MNase),
(f) Adding of a Ca²⁺ ions-containing buffer to start MNase digestion and release of pAG-MNase-antibody-chromatin complexes,
(g) Adding of a chelator-containing buffer to stop the reaction of step (f),
(h) Pelletizing the obtained chromatin fragments and obtaining pAG-MNase-bound digested chromatin fragments from the supernatant,
(i) Extracting of DNA and RNA, respectively, from the chromatin fragments of step (h),
(j) High-throughput sequencing of DNA and RNA, respectively,
(k) Bioinformatic identification of differential peaks of sequencing reads in samples prepared using the catalytically inactive Cas protein (dCas) or catalytically active Cas proteins (Cas)

4. A method to validate CRISPR-Cas binding and cleavage sites comprising the following steps:
(a) Expressing a catalytically inactive Cas protein (dCas) or catalytically active Cas protein containing a protein tag and an sgRNA in target cells,
(b) Optionally hypotonic lysis of the cells of step (a) to release nuclei,
(c) Immobilizing whole cells of step (a) or nuclei of step (b) with magnetic beads,
(d) Incubating the product of step (c) with an antibody against the tag of the protein of step (a),
(e) Incubating the product of step (d) with ProteinA-MNase (pAG-MNase),
(f) Adding of a Ca²⁺ ions-containing buffer to start MNase digestion and release of pAG-MNase-antibody-chromatin complexes,
(g) Adding of a chelator-containing buffer to stop the reaction of step (f),
(h) Pelletizing the obtained oligonucleosome and obtaining pAG-MNase-bound digested chromatin fragments from the supernatant,
(i) Extracting of DNA and RNA, respectively, from the chromatin fragments of step (h),
(j) High-throughput sequencing of DNA and RNA, respectively,
(k) Bioinformatic identification of differential peaks of sequencing reads in samples prepared using the catalytically inactive Cas protein (dCas) or catalytically active Cas proteins (Cas)

5. The method of claim 1, 2, 3, or 4, wherein in step (e) the pAG-MNase is contained in a digitonin-containing buffer.

6. A method to comprehensively capture and analyze CRISPR-Cas binding and cleavage sites comprising the following steps
(a) Expressing a catalytically inactive Cas protein (dCas) or catalytically active Cas proteins (Cas) and a single or several sgRNA in target cells,
(b) Optionally hypotonic lysis of the cells of step (a) to release nuclei,
(c) Immobilizing whole cells of step (a) or nuclei of step (b) with magnetic beads,
(d) Incubating the product of step (c) with an anti-dCas antibody,
(e) Incubating the product of step (d) with a secondary antibody against the the anti-CRISPR-dCas antibody,
(f) Incubating the product of step (d) with a transposome comprising a protein A and/or protein G hyperactive Tn5 fusion protein (pAG-Tn5) loaded with DNA primers duplexes for high-throughput sequencing,
(g) Adding of a Ca²⁺ ions-containing buffer to start tagmentation and release of pAG-Tn5-chromatin complexes,
(h) Adding of a chelator-containing buffer to stop the reaction of step (g),
(i) Pelletizing the obtained oligonucleosome and obtaining pAG-Tn5 bound digested chromatin fragments from the supernatant,
(j) Extracting of DNA from the chromatin fragments of step (i),
(k) High-throughput sequencing of DNA,
(l) Bioinformatic identification of differential peaks of sequencing reads in samples prepared using the catalytically inactive Cas protein (dCas) or catalytically active Cas proteins (Cas)

7. The method of claim 6, wherein in step (a) 3' repair exonuclease 2 (Trex2) is added.

8. A method to comprehensively capture and analyze CRISPR-Cas binding and cleavage sites independently of an sgRNA comprising the following steps:
(a) Expressing a catalytically inactive Cas protein (dCas) or catalytically active Cas proteins (Cas) in target cells,
(b) Optionally hypotonic lysis of the cells of step (a) to release nuclei,
(c) Immobilizing whole cells of step (a) or nuclei of step (b) with magnetic beads,
(d) Incubating the product of step (c) with an anti-dCas antibody,
(e) Incubating the product of step (d) with a secondary antibody against the the anti-CRISPR-dCas antibody,
(f) Incubating the product of step (d) with a transposome comprising a protein A and/or protein G hyperactive Tn5 fusion protein (pAG-Tn5) loaded with DNA primers duplexes for high-throughput sequencing,
(g) Adding of a Ca²⁺ ions-containing buffer to start tagmentation and release of pAG-Tn5-chromatin complexes,
(h) Adding of a chelator-containing buffer to stop the reaction of step (g),
(i) Pelletizing the obtained oligonucleosome and obtaining pAG-Tn5 bound digested chromatin fragments from the supernatant,
(j) Extracting of DNA from the chromatin fragments of step (i),
(k) High-throughput sequencing of DNA,
(l) Bioinformatic identification of differential peaks of sequencing reads in samples prepared using the catalytically inactive Cas protein (dCas) or catalytically active Cas proteins (Cas)

9. A method to validate CRISPR-Cas targeting comprising the following steps:
(a) expressing a catalytically inactive Cas protein (dCas) or catalytically active Cas proteins (Cas) containing a protein tag and a single or several sgRNA in target cells,
(b) optionally hypotonic lysis of the cells of step (a) to release nuclei,
(c) Immobilizing whole cells of step (a) or nuclei of step (b) with magnetic beads,
(d) Incubating the product of step (c) with an antibody against the tag of the protein of step (a),
(e) Incubating the product of step (d) with a secondary antibody against the the anti-tag antibody.
(f) Incubating the product of step (d) with a transposome comprising a protein A and/or protein G hyperactive Tn5 fusion protein loaded with DNA primers duplexes for high-throughput sequencing.
(g) Adding of a Ca²⁺ ions-containing buffer to start tagmentation and release of pAG-Tn5-chromatin complexes,
(h) Adding of a chelator-containing buffer to stop the reaction of step (f),
(i) Pelletizing the obtained oligonucleosome and obtaining pAG-MNase-bound digested chromatin fragments from the supernatant,
(j) Extracting of DNA from the chromatin fragments of step (h),
(l) High-throughput sequencing of DNA,
(l) bioinformatic identification of differential peaks of sequencing reads in samples prepared using the catalytically inactive Cas protein (dCas) or catalytically active Cas proteins (Cas)

10. The method of any of claims 1 to 9, wherein the protein is Cas9 or dCas9 or Cas12 or dCas12.

11. The method of any of claims 1 to 5, wherein the protein is Cas13 or dCas13.

12. The method of any of claims 1 to 11, wherein the optionally present hypotonic lysis step (b) is carried out in a HEPES-buffer containing spermidine.

13. The method of any of claims 1 to 12, wherein the magnetic beads in step (c) are Concanavalin A beads and/or the chelator in step (g) is ethyleneglycol-bis(β-aminoethyl)-N,N,N',N'-tetraacetic acid (EGTA).

14. The method of any of claims 1, 2, 3, 5, 6, 7, 8, 10, 12, or 13 wherein the anti-Cas antibody in step (d) is a rabbit polyclonal anti-Cas9 antibody or mouse monoclonal anti-CRISPR-Cas9 antibody.

15. The method of claim 6, 7, 8, 9, 10, 12, 13, or 14 wherein in step (f) the transposome is contained in a digitonin-containing buffer.
